# FASCICULE DE BREVET EUROPEEN

(11) **EP 0 463 969 B1**
(45) Date de publication et mention de la délivrance du brevet: **08.02.1995**
(21) Numéro de dépôt: 91401739.7
(22) Date de dépôt: 27.06.1991
(51) Int. Cl.: C07D 307/81, C07D 333/28, C07D 233/64, C07D 333/60, C07D 405/04, C07D 403/04, A61K 31/34, A61K 31/38, A61K 31/415

(54) **Nouveau composés de l'acide 4-amino butyrique leur procédé de préparation et les préparations pharmaceutiques qui les contiennent**
Neue 4-Aminobuttersäurederivate, Verfahren zu ihrer Herstellung und diese enthaltende Arzneizubereitungen
New compounds of 4-aminobutyric acid, process for their preparation and pharmaceutical preparations containing them

(30) Priorité: 27.06.1990 FR 9008093
(43) Date de publication de la demande: 02.01.1992
(73) Titulaire: ADIR ET COMPAGNIE, 92415 Courbevoie Cédex (FR)
(72) Inventeur: Debaert, Michel, F-59800 (FR); Berthelot, Pascal, F-59320 Haubordin (FR); Vaccher, Claude, F-59139 Wattignies (FR)

(56) Documents cités:
- EP-A- 0 023 192
- JOURNAL OF ORGANIC CHEMISTRY, vol. 55, no. 10, 11 mai 1990, pages 3088-3097, Washington, DC, US; R.C. PETTER et al.: "Inhibition of gamma-butyrobetaine hydroxylase by cyclopropyl-substituted gamma-butyrobetaines"
- CHEMICAL ABSTRACTS, vol. 73, no. 15, 12 octobre 1970, page 407, résumé no. 77617w, Columbus, Ohio, US; & JP-A-70 016 692 (DAIICHI SEIYAKU) 10-06-1970
- CHEMICAL ABSTRACTS, vol. 82, no. 23, 9 juin 1975, page 526, résumé no. 155373r, Columbus, Ohio, US; && JP-A-7 440 460 (DAIICHI SEIYAKU) 02-11-1974
- CHEMICAL ABSTRACTS, vol. 54, no. 12, 25 juin 1960, colonne 12107f, Columbus, Ohio, US; V.V. PEREKALIN et al.: "Synthesis of gamma-amino acids and pyrrolidones", & ZHUR. OBSHCHEI KHIM. 29, 2905-10 (1959)
- JOURNAL OF MEDICINAL CHEMISTRY, vol. 30, no. 4, avril 1987, pages 743-746, Washington, DC, US; P. BERTHELOT et al.: "Synthesis and pharmacological evaluation of gamma-aminobutyric acid analogues. New ligand for GABA-B sites"
- JOURNAL OF MEDICINAL CHEMISTRY, vol. 25, no. 6, juin 1982, pages 723-730, Washington, DC, US; M.E. SAFDY et al.: "Tryptophan analogues. 1. Synthesis and antihypertensive activity of positional isomers"
- EUROPEAN JOURNAL OF MEDICINAL CHEMISTRY, vol. 18, no. 4, 1983, pages 353-358, Paris, FR; J. MAILLARD et al.: "Synthèse d'amino-2(benzofuryl-2)-4 delta-1 pyrrolines et étude de leurs propriétés antidysrythmiques"

## Description

L'invention concerne de nouveaux composés de l'acide 4 - amino butyrique, leur procédé de préparation et les préparations pharmaceutiques qui les contiennent.

On connait dans la littérature de nombreux composés arylpropioniques, qui possèdent notamment des propriétés analgésiques et anti-inflammatoires. On connait également le Baclofène ou acide 4-amino 3-(4-chlorophényl) butyrique, composé agoniste des récepteurs GABA_{B}, utilisé en thérapeutique humaine pour ses propriétés antispastiques. D'autres acides 4-amino 3-aryl butyrique, notamment des composés hétéroaryl butyriques ont également été décrits (J. Med. Chem. 1987, 30, 743 - 746), montrant une affinité pour le récepteur GABA_{B}. La 4-(benzofuryl-2) pyrrolidinone-2 a été décrite dans la publication European Journal of Medicinal Chemistry, vol. 18, N°4, 1983, pp 355 et 357, comme possédant une activité antidysrythmique. De même, des dérivés pyrrolidinoniques de l'acide γ aminobutyrique sont décrits sans mentionner aucune activité pharmacologique dans les Chemical Abstracts, vol. 82, N°23, 1975, p 526, résumé N°155373r et vol. 73, 1970, p 407, résumé N°77617w. L'acide 3-(2-thiényl) 4-aminobutyrique et l'acide 3-(2-furyl) 4-aminobutyrique sont décrits sans mentionner aucune activité pharmacologique dans le résumé "synthesis of gamma-aminoacides and pyrrolidones" du Chemical Abstracts vol. 54, N°12, 25 juin 1960, colonne 12107f.

On connait également dans l'art antérieur la publication "Journal of Medicinal Chemistry, vol. 25, N°6, juin 1982, pp 723-730" présentant des dérivés de l'acide 4-aminobutyrique substitués en 3 par un radical 2-indolyl décrits uniquement pour leur activité antihypertensive, la demande EP-A-0023192 décrivant des dérivés de l'acide 4-aminobutyrique substitués en 3 par un radical cyclohexyle en tant qu'intermédiaire d'un procédé de synthèse.

La publication "Journal of Organic Chemistry, vol. 55, N° 10, 11 mai 1990, pp 3088-3097" mentionne l'acide (3-cyclopropyl) 4-aminobutanoïque comme inhibiteur d'enzyme.

Les composés de la présente invention possèdent, par rapport aux composés de l'art antérieur, une affinité très sélective et nettement plus importante. Par ailleurs, certains d'entre eux antagonisent l'excitation induite par des convulsivants à des doses inférieures à celle du Baclofène. D'autres possèdent la propriété de stimuler la synthèse d'AMP cyclique au niveau du cortex cérébral, donc d'augmenter les capacités métaboliques du cerveau. L'intensité de cette affinité pour le récepteur GABA_{B} et la puissance de leur activité autorisent, en thérapeutique humaine ou animale, l'administration de doses moindres des composés de l'invention. Cette posologie réduite va de pair avec une diminution des effets secondaires que l'on peut observer avec des composés moins actifs puisqu'il est généralement admis que la toxicité n'est pas reliée au mécanisme d'action pharmacologique, mais qu'elle dépend essentiellement de la structure chimique des composés. Ainsi de faibles quantités des composés de l'invention produisent un effet comparable à celui obtenu avec des quantités beaucoup plus importantes de composés de l'art antérieur ; les risques de manifestations toxiques, en particulier hépatiques sont fortement diminués. Cet avantage est particulièrement intéressant pour les populations fragiles auxquelles les composés s'adressent, généralement des sujets souffrant de troubles spastiques ou des sujets âgés (maladie de Alzheimer, déments séniles ou atteints de troubles de la sénéscence), populations chez lesquelles les fonctions hépatiques sont souvent déjà perturbées.

Plus spécifiquement, l'invention concerne de nouveaux composés de l' acide 4 - amino butyrique répondant à la formule générale (I) : 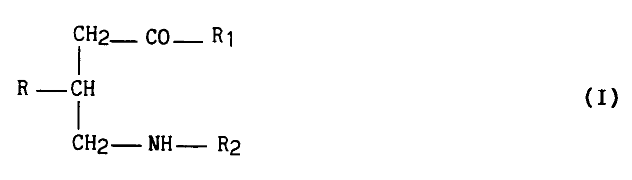
dans laquelle :
- R₁ représente un groupe hydroxy, amino, alkylamino inférieur ou alcoxy inférieur, ou un atome d'halogène,
- R₂ représente un atome d'hydrogène, un radical alkyle inférieur, un radical acyle inférieur, ou un radical alcoxycarbonyle inférieur,
- R représente :
   . un radical de formule : 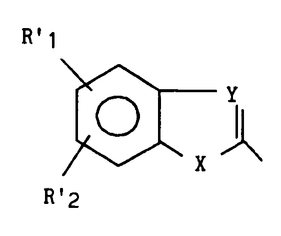 dans laquelle :
      X représente un atome d'oxygène, de soufre, ou un groupement NH,
      Y représente un atome de carbone, d'oxygène, ou d'azote, R'₁ et R'₂ identiques ou différents, représentent un atome d'halogène ou d'hydrogène ou un radical alkyle inférieur ou alcoxy inférieur, hydroxy, nitro, amino, alkylamino inférieur ou trifluorométhyle, avec la réserve que lorsque X est un atome d'oxygène, Y un atome de carbone et R′₁ et R₂ chacun un atome d'hydrogène, alors R′2 ne peut représenter ni un atome d'hydrogène ni un groupe méthoxy,
   . un radical de formule : 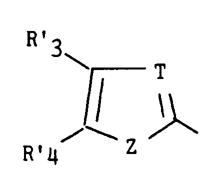 dans laquelle :
      Z représente un atome d'oxygène, de soufre ou un groupement NH,
      T représente un atome de carbone ou d'azote,R′₃ et R′₄ identiques ou différents représentent un radical choisi parmi hydrogène, halogène, alkyle ou alcoxy inférieurs, hydroxy, nitro, amino, alkylamino inférieur ou trifluorométhyle, avec la réserve que lorsque Z est un atome de soufre, T un atome de carbone et R′₃ un atome d'hydrogène, R′₄ ne peut pas être un groupement méthyle ou un atome d'hydrogène, de chlore, ou de brome, et que lorsque Z est un atome d'oxygène, T un atome de carbone et R′₃ un atome d'hydrogène, R′₄ ne peut pas être un atome d'hydrogène ou un groupe méthyle,
   . un radical cycloalkyle de 4 ou 5 atomes de carbone, cycloalkylalkyle ou dicycloalkylalkyle de 4 à 16 atomes de carbone éventuellement substitués sur le cycle par un groupement choisi parmi halogène, hydroxy, alkyle ou alcoxy inférieur, nitro, amino, alkylamino inférieur ou trifluorométhyle,
   . un radical aromatique à 6 sommets éventuellement substitué et incluant dans son squelette carboné 2 ou 3 atomes d'azote,
   . un radical aromatique à 6 sommets incluant dans son squelette carboné de 1 à 3 atomes d'azote et accolé à un cycle benzénique, chacun de ces 2 cycles pouvant être éventuellement substitué,
   . ou un cycle à sept sommets, saturé ou non, incluant dans son squelette carboné un ou deux atomes d'azote éventuellement accolé à un cycle benzénique et éventuellement substitué sur le cycle azoté et/ou benzénique,
le terme substitué signifiant que les groupements qu'il affecte peuvent être substitués par un ou plusieurs atomes d'halogène ou groupements alkyle inférieur ou alcoxy inférieur, ou hydroxy, ou trifluorométhyle, ou nitro, ou amino, ou alkylamino inférieur,
leurs isomères optiques ainsi que le cas échéant leurs sels d'addition à une base ou un acide pharmaceutiquement acceptable,
étant entendu que, sauf précision contraire, les termes "alkyle inférieur", "alcoxycarbonyle inférieur", "alcoxy inférieur", "alkylamino inférieur", et "acyle inférieur" signifient des groupements contenant de 1 à 6 atomes de carbone, en chaine droite ou ramifiée.

Parmi les acides ou les bases pharmaceutiquement acceptables que l'on peut utiliser pour salifier les composés de l'invention, on peut citer, à titre d'exemples non exhaustifs, les acides chlorhydrique, bromhydrique, sulfurique, nitrique, oxalique, malique, maléique, succinique, tartrique, méthanesulfonique, camphorique, camphosulfonique, la soude, la potasse, la triéthylamine, la diéthylamine, l'éthanolamine ou la diéthanolamine, l'arginine, la lysine...

L'invention s'étend aussi au procédé de préparation des composés de formule (I), caractérisé en ce que l'on utilise comme matière première un composé de formule (II) : 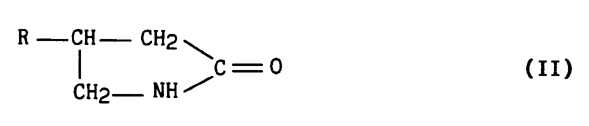
dans laquelle R a la même signification que dans la formule (I),
que l'on peut :
- soit hydrolyser par action d'un hydroxyde métallique pour obtenir après éventuelle purification un composé de formule (I/a) : 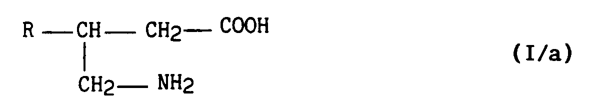 cas particulier des composés de formule (I) pour lesquels R₁ représente un groupe hydroxy, R₂ représente un atome d'hydrogène et R a la même signification que dans la formule (I),
- soit transformer par action d'un dicarbonate d'alkyle inférieur en présence d'une base forte en un composé de formule (III) : 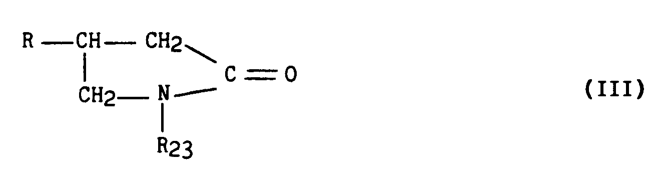 dans laquelle R a la même signification que précédemment, et R₂₃ représente un groupement alcoxycarbonyle inférieur,
   que l'on traite après éventuelle purification par un hydroxyde alcalin en milieu anhydre, puis par un acide pour obtenir un composé de formule (I/b) : 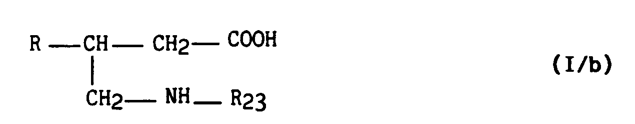 avec R et R₂₃ tels que définis précédemment,
   cas particulier des composés de formule (I) pour lesquels R₁ représente un groupe hydroxy, R₂ représente un radical alcoxycarbonyle inférieur et R a la même signification que dans la formule (I),
   qui, traité en milieu acide, conduit à un dérivé de formule (I/a) tel que défini précédemment,
   composé de formule (I/a) qui, quel que soit le procédé selon lequel il a été obtenu, peut être, si on le désire, transformé par un agent d'halogénation en son halogénure de formule (I/c) : 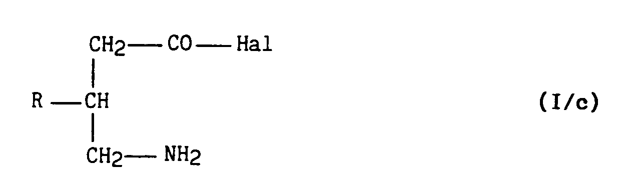 dans laquelle Hal représente un atome d'halogène et R a la même signification que dans la formule (I),
   cas particulier des composés de formule (I) dans laquelle R₁ représente un atome d'halogène, R₂ représente un atome d'hydrogène, et R a la même signification que dans la formule (I),
   composé de formule (I/a) ou (I/c) que l'on peut traiter, si on le désire :
- par un composé de formule :

   **R₁'' - H**

   dans laquelle R₁'' représente un groupement amino, alkylamino inférieur ou alcoxy inférieur,
   pour conduire à un composé de formule (I) pour lequel R₁ représente un groupement amino, alkylamino inférieur ou alcoxy inférieur,
- et, si on le désire, par un agent d'alkylation comme le sulfate de diméthyle ou un halogénure d'alkyle de formule :

   **R₂₁ - X**

   dans laquelle R₂₁ signifie un groupement alkyle inférieur et X représente un atome d'halogène,
   pour conduire à un composé de formule (I), dans lequel R₂ représente un groupement alkyle inférieur,
   ou bien encore, si on le désire, par un chlorure d'acide de formule :

   **R₂₂Cl**

   ou un anhydride d'acide de formule :

   **R₂₂OR₂₂**

   R22 signifiant un groupement acyle inférieur, pour conduire à un composé de formule (I) dans lequel R₂ représente un groupement acyle inférieur,
   les composés de formule (I) étant ensuite, si on le désire, soit dédoublés en leurs isomères optiques puis salifiés par addition d'une base ou d'un acide pharmaceutiquement acceptable, soit salifiés directement sous forme racémique par addition d'une base ou d'un acide pharmaceutiquement acceptable.

Les composés de formule (II) peuvent être obtenus :
- soit par condensation dans un solvant apolaire aprotique d'un aldéhyde de formule (V):

   **R - CHO** **(V)**

   dans laquelle R a la même signification que dans la formule (I),
   avec un ester de carboxyméthylidène triphénylphosphorane de formule (VI) :

   **(C₆H₅)₃ - P = CH - COOR' (VI)**

   dans laquelle R′ représente un radical alkyle inférieur,
   pour obtenir un ester de formule (VII) :

   **R - CH = CH - COOR' (VII)**

   dans laquelle R et R' ont les mêmes significations que précédemment,
   que l'on condense, en milieu polaire protique avec le nitrométhane en présence d'une base forte, pour obtenir un composé de formule (VIII) : 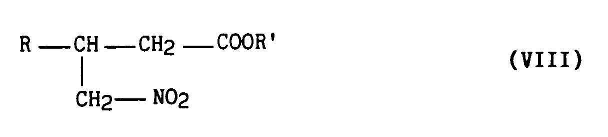 dans laquelle R et R' ont les mêmes significations que précédemment,
   qui est réduit en milieu alcoolique par action de l'hydrogène en présence d'un catalyseur métallique, en un composé de formule (IX) : 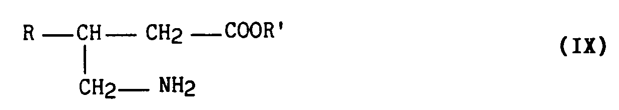 dans laquelle R et R' ont les mêmes significations que précédemment,
   qui est cyclisé par chauffage en un composé de formule (II) : 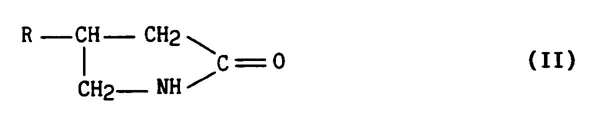 dans laquelle R a la même signification que dans la formule (I),
- soit par traitement d'un composé de formule (X) :

   **RCOCH₃ (X)**

   dans laquelle R a la même définition que dans la formule (I),
   à chaud en présence de zinc par un composé de formule (XI) :

   **Br - CH₂ - COOA (XI)**

   dans laquelle A représente un groupement alkyle inférieur,
   pour conduire, après hydrolyse acide, extraction et purification éventuelles, à un composé de formule (XII) : 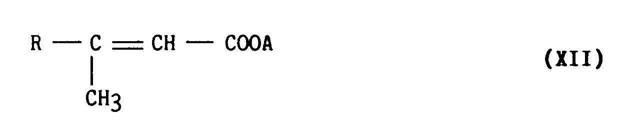 dans laquelle R et A ont la même signification que précédemment,
   que l'on traite par la N bromo succinimide pour conduire à un composé de formule (XIII) : 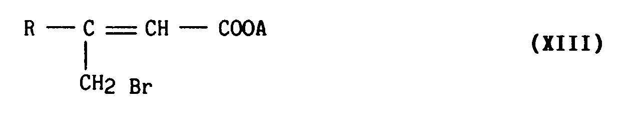 dans laquelle R et A ont la même signification que précédemment,
   que l'on traite par l'ammoniac préférentiellement en excès pour conduire à un composé de formule (XIV) : 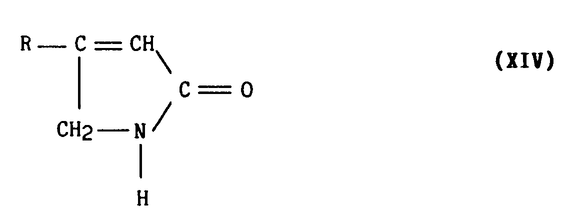 dans lequel R a la même définition que précédemment,
   que l'on soumet à une hydrogénation catalytique pour obtenir un composé de formule (II) tel que ci-dessus défini.

Les composés de formule (II), où R ne représente pas :
- un groupement 2-benzofuryle non substitué ou substitué sur le noyau benzénique par un atome de chlore, un atome de brome, ou un groupe méthoxy,
- ou un groupement 2-benzothiényle, ainsi que les composés de formule (III) sont nouveaux et sont partie intégrante de la présente invention en tant que matière première utile pour réaliser la synthèse des composés de l'invention.

Les composés de formule (I) possèdent des propriétés pharmacologiques intéressantes. Ils possèdent une affinité trés importante et sélective pour le récepteur GABA_{B}, supérieure à celle des dérivés de l'art antérieur.

Certains d'entre eux possèdent une activité antagoniste des récepteurs GABA_{B} et à ce titre peuvent être administrés dans le traitement des troubles de la mémoire, les troubles mentaux de la sénéscence, ainsi que dans le traitement de la maladie d'Alzheimer.

D'autres composés, à l'inverse, présentent une activité agoniste et s'adressent alors à des sujets spastiques ou souffrant d'angine de poitrine.

La présente invention a également pour objet les compositions pharmaceutiques contenant les dérivés de formule (I), ou un de leurs sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, on pourra citer plus particulièrement celles qui conviennent à l'administration orale, parentérale, nasale, rectale, perlinguale, oculaire ou pulmonaire et notamment les préparations injectables, les aérosols, les gouttes oculaires ou nasales, les comprimés simples ou dragéifiés, les comprimés sublinguaux, les gélules, les paquets, les suppositoires, les crèmes, pommades, gels dermiques...

La posologie varie selon l'âge et le poids du patient, la voie d'administration, la nature de l'affection et des traitements éventuellement associés et s'échelonne entre 1 mg et 1 gramme par 24 heures.

Les exemples qui suivent illustrent l'invention et ne la limitent en aucune façon.

Les matières premières de départ sont décrites dans la littérature ou peuvent être préparées d'une manière similaire.

Les spectres de résonnance magnétique nucléaire 1H (RMN) ont été réalisés en utilisant le tétraméthylsilane (TMS) comme référence interne. Les déplacements chimiques sont exprimés en partie par million (ppm).

Les spectres infrarouge ont été effectués sous forme de pastille de bromure de potassium renfermant environ 1 % du procduit à analyser.

### EXEMPLE 1 : Acide 3-(2-(5-isopropyl benzofuryl)) 4-amino butanoïque

### STADE A : 3-(2-(5-isopropyl benzofuryl)) propénoate d'éthyle

Chauffer à reflux sous azote pendant 4 heures une solution contenant 0,1 mole de 2-(5-isopropyl benzofuryl) carbaldéhyde et 0,1 mole de carbéthoxyméthylidène triphénylphosphorane dans 200 cm³ de benzène. Refroidir, évaporer le solvant sous pression réduite. Reprendre le résidu par 200 cm³ d'éther, éliminer l'insoluble par essorage et évaporer à sec le filtrat, puis distiller sous pression réduite. On obtient le composé du stade A :
Eb(3 mm Hg) = 186 °C.

### STADE B : 3-(2-(5-isopropyl benzofuryl)) 4-nitro butanoate d'éthyle

Chauffer à 70 °C pendant 18 heures 0,05 mole du composé obtenu au stade A de l'exemple 1 dans 50 cm³ de nitrométhane et 2 cm³ d'une solution méthanolique de triton B à 40 %. Refroidir, neutraliser par une solution molaire d'acide chlorhydrique et extraire à l'éther. Laver à l'eau la phase éthérée, sécher, filtrer, puis évaporer à sec. Le composé du stade B est obtenu après purification par chromatographie liquide haute performance.

### STADE C : 4-(2-(5-isopropyl benzofuryl)) 2-oxopyrrolidine

Réduire 0,05 mole du composé obtenu au stade B de l'exemple 1 en solution éthanolique, par l'hydrogène à pression atmosphérique et à température ambiante en présence de nickel de Raney. Filtrer, évaporer sous pression réduite, puis chauffer le résidu pendant 2 heures. Recristalliser dans l'éther de pétrole.
Point de fusion : 151 °C
Caractéristiques spectrales :
- infrarouge : 1690 cm⁻¹ : νCO
   3300 m⁻¹ : νNH
- RMN (CDCl₃) :
   δ : 1,26 ppm : doublet : ((CH₃)₂C)
   δ : 2,75 ppm : doublet : (CH₂ CO)
   δ : 2,80 - 3,20 ppm : multiplet : (CH (CH₃)₂)
   δ : 3,40 - 4,10 ppm : multiplet : (CH, CH₂ N)
   δ : 5,70 ppm : singulet : (NH)
   δ : 6,50 ppm : singulet : (H₃' ; benzofurane)
   δ : 7,00 - 7,50 ppm : multiplet : (H₄', H₆', H₇' ; benzofurane)

### STADE D : acide 3-(2-(5-isopropyl bensofuryl)) 4-amino butanoïque

Chauffer pendant une heure à reflux 0,01 mole de composé obtenu au stade C de l'exemple 1, dans 20 cm³ d'éthanol à 95° en présence de 5 cm³ de soude à 40 %. Refroidir, évaporer à sec. Reprendre le résidu par 15 à 20 cm³ d'eau, acidifiée jusqu'à pH = 1 par HCl à 10 %.
Evaporer à sec, reprendre par 1 à 3 cm³ d'acide trifluoracétique et chromatographier sur résine échangeuse d'ions (DOWEX 50 WH+) en éluant par une solution d'amoniaque à 5 %. Evaporer à sec l'éluant et recristalliser le résidu dans l'éthanol.
Point de fusion : 190 °C
Caractéristiques spectrales :
- infrarouge : 1580 cm⁻¹ : νCO
   2300 - 3200 cm⁻¹ : νOH

### EXEMPLE 2 : Acide 3-(2-(5-méthyl benzofuryl) 4-amino butanoïque

En suivant les instructions des stades A à D de l'exemple 1 mais en remplaçant au stade A de l'exemple 1 le 2-(5-isopropyl benzofuryl) carbaldéhyde par le 2-(5-méthyl benzofuryl) carbaldéhyde, on obtient successivement, après une éventuelle étape de purification par chromatographie liquide haute performance, les composés suivants :

### STADE A : 3-(2-(5-méthyl benzofuryl)) propénoate d'éthyle

Point de fusion : 73 °C

### STADE B : 3-(2-(5-méthyl benzofuryl)) 4-nitro butanoate d'éthyle

### STADE C : 4-(2-(5-méthyl benzofuryl)) 2-oxopyrrolidine

Point de fusion : 151 °C
Caractéristiques spectrales :
- infrarouge : 1690 cm⁻¹ : νCO
   3300 m⁻¹ : νNH
- RMN (CDCl₃) :
   δ : 2,41 ppm : singulet : (CH₃)
   δ : 2,69 ppm : doublet : (CH₂CO)
   δ : 3,50 - 4,10 ppm : multiplet : (CH₂N, CH)
   δ : 6,00 ppm : singulet : (NH)
   δ : 6,46 ppm : singulet : (H₃', benzofurane)
   δ : 6,90 - 7,50 ppm : multiplet : (H₄', H₆', H₇' ; benzofurane)

### STADE D : acide 3-(2-(5-méthyl benzofuryl)) 4-amino-butanoïque

Point de fusion : 191 °C
Caractéristiques spectrales :
- infrarouge : 1580 cm⁻¹ : νCO
   2300 - 3200 cm⁻¹ : ν(COO⁻, NH₃⁺)

### EXEMPLE 3 : Acide 3-(2-(5-éthyl benzofuryl)) 4-amino butanoïque

En suivant les instructions des stades A à D de l'exemple 1 mais en remplaçant au stade A de l'exemple 1 le 2-(5-isopropyl benzofuryl) carbaldéhyde par le 2-(5-éthyl benzofuryl) carbaldéhyde, on obtient successivement, après une éventuelle étape de purification par chromatographie liquide haute performance, les composés suivants :

### STADE A : 3-(2-(5-éthyl benzofuryl)) propénoate d'éthyle

Point de fusion : 52 °C

### STADE B : 3-(2-(5-éthyl benzofuryl)) 4-nitro butanoate d'éthyle

### STADE C : 4-(2-(5-éthyl benzofuryl)) 2-oxopyrrolidine

Point de fusion : 125 - 127 °C
Caractéristiques spectrales :
- infrarouge : 1670 cm⁻¹ : νCO
   3200 m⁻¹ : νNH
- RMN (CDCl₃) :
   δ : 1,25 ppm : triplet : (CH₂ - CH₃)
   δ : 2,50 - 3,00 ppm : multiplet : (CH₂ - CH₃, CH₂CO)
   δ : 3,40 - 4,10 ppm : multiplet : (CH₂N, CH)
   δ : 5,93 ppm : singulet : (NH)
   δ : 6,46 ppm : singulet : (H₃', benzofurane)
   δ : 7,00 - 8,00 ppm : multiplet : (H₄', H₆', H₇' ; benzofurane)

### STADE D : acide 3-(2-(5-éthyl benzofuryl)) 4-amino butanoïque

Point de fusion : 195 °C
Caractéristiques spectrales :
- infrarouge : 1580 cm⁻¹ : νCO
   2300 - 3200 cm⁻¹ : νOH

### EXEMPLE 4 : Acide 3-(2-(5-(1-méthylpropyl) benzofuryl)) 4-amino butanoïque

En suivant les instructions des stades A à D de l'exemple 1 mais en remplaçant au stade A de l'exemple 1 le 2-(5-isopropyl benzofuryl) carbaldéhyde par le 2-(5-(1-méthylpropyl) benzofuryl) carbaldéhyde, on obtient successivement, après une éventuelle étape de purification par chromatographie liquide haute performance, les composés suivants :

### STADE A : 3-(2-(5-(1-méthylpropyl) benzofuryl)) propénoate d'éthyle

Eb (0,5 mm Hg) = 165 °C

### STADE B : 3-(2-(5-(1-méthylpropyl) benzofuryl)) 4-nitro butanoate d'éthyle

### STADE C : 4-(2-(5-(1-méthylpropyl) benzofuryl)) 2-oxopyrrolidine

Point de fusion : 111 - 113 °C
Caractéristiques spectrales :
- infrarouge : 1700 cm⁻¹ : νCO
   3200 m⁻¹ : νNH
- RMN (CDCl₃) :
   δ : 0,80 ppm : triplet : (CH₃ - CH₂ -)
   δ : 1,28 ppm : doublet : (CH₃ - CH -)
   δ : 1,45 - 1,80 ppm : multiplet : (- CH₂ - CH)
   δ : 2,68 ppm : doublet : (CH₂CO)
   δ : 3,50 - 4,10 ppm : multiplet : (CH₂N, CH)
   δ : 5,66 ppm : singulet : (NH)
   δ : 6,49 ppm : singulet : (H₃', benzofurane))
   δ : 7,00 - 7,50 ppm : multiplet : (H₄', H₆', H₇' ; benzofurane)

### STADE D : acide 3-(2-(5-(1-méthylpropyl) benzofuryl)) 4-amino butanoïque

Point de fusion : 200 °C
Caractéristiques spectrales :
- infrarouge : 1580 cm⁻¹ : νCO
   2300 - 3200 cm⁻¹ : νOH

### EXEMPLE 5 : Acide 3-(2-(5-fluoro benzofuryl)) 4-amino butanoïque

En suivant les instructions des stades A à D de l'exemple 1 mais en remplaçant au stade A de l'exemple 1 le 2-(5-isopropyl benzofuryl) carbaldéhyde par le 2-(5-fluoro benzofuryl) carbaldéhyde, on obtient successivement, après une éventuelle étape de purification par chromatographie liquide haute performance, les composés suivants :

### STADE A : 3-(2-(5-fluoro benzofuryl)) propénoate d'éthyle

Point de fusion : 112 °C

### STADE B : 3-(2-(5-fluoro benzofuryl)) 4-nitro butanoate d'éthyle

### STADE C : 4-(2-(5-fluoro benzofuryl)) 2-oxopyrrolidine

Point de fusion : 178 - 180 °C
Caractéristiques spectrales :
- infrarouge : 1690 cm⁻¹ : νCO
   3200 m⁻¹ : νNH
- RMN (CDCl₃) :
   δ : 2,70 ppm : doublet : (CH₂ CO)
   δ : 3,40 - 4,10 ppm : multiplet : (CH₂N, CH)
   δ : 5,75 ppm : singulet : (NH)
   δ : 6,50 ppm : singulet : (H₃' ; benzofurane)
   δ : 6,80 - 7,50 ppm : multiplet : (H₄', H₆', H₇' ; benzofurane)

### STADE D : acide 3-(2-(5-fluoro benzofuryl)) 4-amino butanoïque

Point de fusion : 200 - 202 °C
Caractéristiques spectrales :
- infrarouge : 1580 cm⁻¹ : νCO
   2300 - 3200 cm⁻¹ : νOH
- RMN (D₂O) :
   δ : 2,76 ppm : doublet : (CH₂ CO)
   δ : 3,30 - 4,00 ppm : multiplet : (CH₂N, CH)
   δ : 6,88 ppm : singulet : (H₃' ; benzofurane)
   δ : 6,90 - 7,90 ppm : multiplet : (H₄', H₆', H₇' ; benzofurane)

### EXEMPLE 6 : Acide 3-(2-(5-bromo benzofuryl)) 4-amino butanoïque

En suivant les instructions des stades A à D de l'exemple 1 mais en remplaçant au stade A de l'exemple 1 le 2-(5-isopropyl benzofuryl) carbaldéhyde par le 2-(5-bromo benzofuryl) carbaldéhyde, on obtient successivement, après une éventuelle étape de purification par chromatographie liquide haute performance, les composés suivants :

### STADE A : 3-(2-(5-bromo benzofuryl)) propénoate d'éthyle

### STADE B : 3-(2-(5-bromo benzofuryl)) 4-nitro butanoate d'éthyle

### STADE C : 4-(2-(5-bromo benzofuryl)) 2-oxopyrrolidine

### STADE D : acide 3-(2-(5-bromo benzofuryl)) 4-amino butanoïque

Point de fusion : 200 - 202 °C
Caractéristiques spectrales :
- infrarouge : 1580 cm⁻¹ : νCO
   2300 - 3200 cm⁻¹ : νOH
- RMN (D₂O) :
   δ : 2,76 ppm : doublet : (CH₂ CO)
   δ : 3,30 - 4,00 ppm : multiplet : (CH₂N, CH)
   δ : 6,88 ppm : singulet : (H₃' ; benzofurane)
   δ : 6,90 - 7,90 ppm : multiplet : (H₄', H₆', H₇' ; benzofurane)

### EXEMPLE 7 : Acide 3-(2-(4,5-dichloro thiényl)) 4-amino butanoïque

En suivant les instructions des stades A à D de l'exemple 1 mais en remplaçant au stade A de l'exemple 1 le 2-(5-isopropyl benzofuryl) carbaldéhyde par le 2-(4,5-dichloro thiényl) carbaldéhyde, on obtient, après une éventuelle étape de purification par chromatographie liquide haute performance, les composés suivants :

### STADE A : 3-(2-(4,5-dichloro thiényl)) propénoate d'éthyle

Point de fusion : 76 - 78 °C

### STADE B : 3-(2-(4,5-dichloro thiényl)) 4-nitro butanoate d'éthyle

### STADE C : 4-(2-(4,5-dichloro thiényl)) 2-oxopyrrolidine

### STADE D : acide 3-(2-(4,5-dichloro thiényl)) 4-amino butanoïque

Point de fusion : 188 - 195 °C
Caractéristiques spectrales :
- infrarouge : 2500 - 3400 cm⁻¹ : large bande (NH₃⁺, COO⁻)
   1590 cm⁻¹ : νCO
- RMN (D₂O) :
   δ : 2,6 ppm : doublet : (CH₂ CO)
   δ : 3,1 - 3,5 ppm : multiplet : 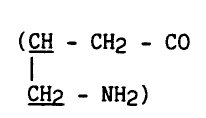 δ : 7 ppm : singulet : (H ; thiophène)

### EXEMPLE 8 : Acide 3-(2-imidazolyl) 4-amino butanoïque

En suivant les instructions des stades A à D de l'exemple 1 mais en remplaçant au stade A de l'exemple 1 le 2-(5-isopropyl benzofuryl) carbaldéhyde par le 2-imidazolyl carbaldéhyde, on obtient, après une éventuelle étape de purification par chromatographie liquide haute performance, les composés suivants :

### STADE A : 3-(2-imidazolyl) propénoate d'éthyle

### STADE B : 3-(2-imidazolyl) 4-nitro butanoate d'éthyle

### STADE C : 4-(2-imidazolyl) 2-oxopyrrolidine

Caractéristiques spectrales :
- infrarouge : 3200 - 3100 cm⁻¹ : νNH
   1700 cm⁻¹ : νCO
- RMN (DMSO) :
   δ : 2,5 ppm : doublet : (CH₂ CO)
   δ : 3,5 ppm : multiplet : 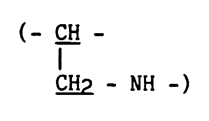 δ : 6,9 ppm : singulet : (H₂' ; imidazole)
   δ : 7,5 ppm : singulet : (NH - CO)
   δ : 10 - 11 ppm : singulet : (NH ; imidazole)

### STADE D : acide 3-(2-imidazolyl) 4-amino butanoïque

Point de fusion : 175 - 180 °C
Caractéristiques spectrales :
- infrarouge : 2700 - 3400 cm⁻¹ : ν(COO-,NH₃⁺)
   1590 cm⁻¹ : νCO
- RMN (D₂O) :
   δ : 2,6 ppm : doublet : (CH₂ CO)
   δ : 3,2 - 3,7 ppm : multiplet : (CH - CH₂ - NH)
   δ : 7,05 ppm : singulet : (H₂' ; imidazole)

### EXEMPLE 9 : Acide 3-(2-(5-chloro benzofuryl)) 4-amino butanoïque

En suivant les instructions des stades A à C de l'exemple 1 mais en remplaçant au stade A de l'exemple 1 le 2-(5-isopropyl benzofuryl) carbaldéhyde par le 2-(5-chloro benzofuryl) carbaldéhyde, on obtient, successivement, les composés suivants :

### STADE A : le 3-(2-(5-chloro benzofuryl)) propénoate d'éthyle

### STADE B : le 3-(2-(5-chloro benzofuryl)) 4-nitrobutanoate d'éthyle

### STADE C : le 4-(2-(5-chloro benzofuryl)) 2-oxopyrrolidine

### STADE D : 1-tert.butoxycarbonyl 2-oxo 4-(2-(5-chloro benzofuryl)) pyrrolidine

Sous atmosphère d'azote et à température ambiante, ajouter à 0,01 mole du composé obtenu au stade C de l'exemple 9 en solution dans 50 cm³ de chlorure de méthylène, 0,01 mole de triéthylamine, 0.02 mole de dicarbonate de ditert.butyle et 0,01 mole de diméthylaminopyridine. Agiter le mélange réactionnel pendant 7 heures, évaporer à sec, reprendre le résidu obtenu par 25 cm³ d'éther et éliminer le précipité formé par filtration. Laver la phase éthérée à l'eau, sécher et recristalliser dans l'éther isopropylique.
Point de fusion : 123 °C
Caractéristiques spectrales :
- infrarouge : 3100 cm⁻¹ : νNH
   1800 cm⁻¹ : νCO (tert.butyloxycarbonyl)
   1690cm⁻¹ : νCO (lactame)
- RMN (CDCl₃) :
   δ : 1,5 ppm : singulet : (tert.butyloxycarbonyl)
   δ : 2,9 ppm : doublet : (CH₂ - CO)
   δ : 4 ppm : multiplet : (CH - CH₂ - N)
   δ : 6,5 ppm : singulet : (H₃' ; benzofurane)
   δ : 7,15 ppm : doublet : (H₆' ; benzofurane)
   δ : 7,25 ppm : doublet : (H₇' ; benzofurane)
   δ : 7,5 ppm : doublet : (H₄' ; benzofurane)

### STADE E : acide 4-tert.butyloxycarbonylamino 3-(2-(5-chloro benzofuryl)) butanoïque

A 0,01 mole du composé obtenu au stade D de l'exemple 9, en solution dans le tétrahydrofurane, ajouter, à température ambiante, 0,01 mole d'une solution molaire d'hydroxyde de lithium. Agiter pendant trente minutes, évaporer le solvant, reprendre par 25 cm³ d'eau, puis acidifier par une solution d'acide acétique à 10 %. Extraire à l'éther, sécher, filtrer, évaporer et recristalliser dans l'hexane.
F °C : 104 °C

### STADE F : acide 3-(2-(5-chloro benzofuryl)) 4-amino butanoïque

Agiter pendant une heure à température ambiante un mélange de 0,005 mole du composé obtenu au stade E de l'exemple 9 et de 0,25 mole d'acide trifluoracétique dans 100 cm³ de dichlorométhane, évaporer à sec, reprendre par 25 cm³ d'eau puis acidifier jusqu'à pH = 1 par une solution d'acide chlorhydrique à 10 %, reprendre par 1 à 3 cm³ d'acide trifluoracétique, chromatographier sur résine échangeuse d'ions (DOWEX 50 WH+) en éluant par une solution d'ammoniaque à 5 %. Evaporer à sec l'éluant et recristalliser le résidu dans l'eau.
- F °C: : 190 - 192 ° C
- IR: : 2300 - 3300 cm⁻¹ : ν (COO⁻, NH₃⁺)
1590 cm⁻¹ : ν CO
- RMN (D₂O): : δ : 2,75 ppm : doublet, 2H : (CH2 - COOH)
δ : 3,25 - 4 ppm : multiplet - 3H : (CH - CH₂ - NH₂)
δ : 6,8 ppm : singulet - 1H : (furanne)
δ : 7,25 - 7,75 ppm : multiplet - 3H : (benzène)

### EXEMPLE 10 : Acide 3-(2-benzothiényl) 4-amino butanoïque

En procédant comme dans l'exemple 9, mais en utilisant initialement au stade A de l'exemple 9 le 2-benzothiényl carbaldéhyde, on obtient :

### STADE D : 1-tert.butyloxycarbonyl 2-oxo 4-(2-benzothiényl) pyrrolidine

et au

### STADE F : le produit du titre :

- F °C: : 192 - 197 ° C
- IR: : 2 300 - 3 200 cm⁻¹ : ν (COO⁻, NH₃⁺)
1 575 cm⁻¹ : ν CO
- RMN (D₂O): : δ : 2,7 ppm : doublet - 2H : (CH2 - CO)
δ : 3,3 - 3,5 ppm : multiplet - 2H : (CH₂ - NH₂)
δ : 3,75 ppm : multiplet - 1H : (CH)
δ : 7,25 - 8,10 ppm : multiplet - 5H : (benzothiophène)

### EXEMPLE 11 : Acide 3-(2-(5-éthoxy benzofuryl)) 4-amino butanoïque

En procédant comme dans l'exemple 9, mais en utilisant initialement au stade A de l'exemple 9 le 2-(5-éthoxy benzofuryl) carbaldéhyde, on obtient :

### STADE C : 4-(2-(5-éthoxy benzofuryl)) 2-oxopyrrolidine

Point de fusion : 146 - 148 °C
Caractéristiques spectrales :
- infrarouge : 1675 cm⁻¹ : νCO
   3250 cm⁻¹ : νNH
- RMN(CDCl₃) :
   δ : 1,35 ppm : triplet : (O - CH₂ - CH₃)
   δ : 2,50 - 2,70 ppm : doublet : (CH₂, CO)
   δ : 3,50 - 4,10 ppm : multiplet : (CH₂ N, CH, O - CH₂ - CH₃)
   δ : 6,15 ppm : singulet : (NH)
   δ : 6,47 ppm : singulet : (H₃' ; benzofurane)
   δ : 6,85 ppm : doublet : (H₄' ; benzofurane)
   δ : 7,00 ppm : doublet : (H₆' ; benzofurane)
   δ : 7,30 ppm : doublet : (H₇' ; benzofurane)

### STADE D : 1-tert.butyloxycarbonyl 2-oxo 4-(2-(5-éthoxy benzofuryl)) pyrrolidine

et au

### STADE F : le produit du titre

Point de fusion : 200 °C Caractéristiques spectrales :
- infrarouge : 2300 - 3200 cm⁻¹ : ν(COO⁻, NH₃⁺)
   1620 cm⁻¹ : ν CO
- RMN (D₂O) :
   δ : 1,40 ppm : triplet : (CH₃)
   δ : 2,60 : doublet : (CH₂CO)
   δ : 3,30 - 3,95 ppm : multiplet : (CH - CH₂ - NH₂)
   δ : 4,10 ppm : quintuplet : (OCH₂)
   δ : 6,75 ppm : singulet : (H₃' ; benzofurane)
   δ : 7,00 ppm : doublet : (H₆' ; benzofurane)
   δ : 7,20 ppm : doublet : (H₄' ; benzofurane)

### EXEMPLE 12 : Acide 3-(2-benzothiazolyl) 4-amino butanoïque

En procédant comme dans l'exemple 9, mais en utilisant initialement au stade A de l'exemple 9 le 2-benzothiazolyl carbaldéhyde, on obtient :

### STADE C : 4-(2-benzothiazolyl) 2-oxopyrrolidine

### STADE D : 1-tert.butyloxycarbonyl 2-oxo 4-(2-benzothiazolyl) pyrrolidine

et au

### STADE F : le produit du titre

### EXEMPLE 13 : Acide 3-(2-(5-trifluorométhyl benzofuryl)) 4-amino butanoïque

En procédant comme dans l'exemple 9, mais en utilisant initialement au stade A de l'exemple 9 le 2-(5-trifluorométhyl benzofuryl) carbaldéhyde, on obtient :

### STADE C : 4-(2-(5-trifluorométhyl benzofuryl)) 2-oxopyrrolidine

### STADE D : 1-tert.butyloxycarbonyl 2-oxo 4-(2-(5-trifluorométhyl benzofuryl)) pyrrolidine

et au

### STADE F : le produit du titre

### EXEMPLE 14 : Acide 3-(2-(4-trifluorométhyl thiényl)) 4-amino butanoïque

En procédant comme dans l'exemple 9, mais en utilisant initialement au stade A de l'exemple 9 le 2-(4-trifluorométhyl thiényl) carbaldéhyde, on obtient :

### STADE C : 4-(2-(4-trifluorométhyl thiényl)) 2-oxopyrrolidine

### STADE D : 1-tert.butyloxycarbonyl 2-oxo 4-(2-(5-trifluorométhyl thiényl)) pyrrolidine

et au

### STADE F : le produit du titre

### EXEMPLE 15 : Acide 3-dicyclopropylmethyl 4-amino butanoïque

En procédant comme dans l'exemple 9, mais en utilisant initialement au stade A de l'exemple 9 le 2-dicyclopropylméthyl carbaldéhyde, on obtient :

### STADE C : 4-dicyclopropylméthyl 2-oxopyrrolidine

### STADE D : 1-tert.butyloxycarbonyl 2-oxo 4-dicyclopropylméthyl pyrrolidine

et au

### STADE F : le produit du titre

### EXEMPLE 16 : Ester méthylique de l'acide 3 - (2-imidazolyl) 4-amino butanoïque

### STADE A : chlorure de l'acide 3-(2-imidazolyl) 4-amino butanoïque

Dissoudre 0,01 mole du chlorure d'acide 3-(2-imidazolyl) 4-aminobutyrique dans 30 cm³ de chlorure de méthylène, ajouter 0,025 mole de chlorure de thionyle et agiter à température ambiante pendant 2 heures. Evaporer le milieu réactionnel et extraire à deux reprises au chloroforme après alcalinisation. Réunir les phases chloroformiques, sécher sur chlorure de calcium et évaporer le chloroforme. Recristalliser le résidu.

### STADE B : ester méthylique de l'acide 3-(2-imidazolyl) 4 - amino butanoïque

Dissoudre 0,01 mole du composé obtenu au stade A de l'exemple 16 dans 30 cm³ de pyridine. Ajouter 1 cm³ de méthanol. Porter à reflux cinq heures, évaporer le milieu réactionnel au bain-marie sous vide. Sécher. Purifier par chromatographie.

### EXEMPLE 17 : 3 - (2-imidazolyl) 4 - amino butyramide

Dissoudre 0,01 mole du chlorure de l'acide 3 - (2-imidazolyl) 4 - amino butanoïque obtenu exemple 12, stade A, dans 50 cm³ de chloroforme. Faire barboter un courant d'ammoniac pendant 2 heures. Evaporer à sec le milieu réactionnel et recristalliser le résidu.

### EXEMPLE 18 : 3 - (2-imidazolyl) 4 - amino N propyl butyramide

Dissoudre 0,01 mole du composé obtenu au stade A de l'exemple 16 dans 50 cm³ de chloroforme. Ajouter 0,025 mole de propylamine et porter à reflux 5 heures sous agitation. Refroidir. Evaporer à sec et extraire au chloroforme à deux reprises. Réunir les phases chloroformiques, sécher sur chlorure de calcium et évaporer au bain-marie sous vide. Purifier le résidu par chromatographie.

### EXEMPLE 19 : Ester méthylique de l'acide 3-(2-(5-isopropyl benzofuryl)) 4-amino butanoïque

En procédant comme dans l'exemple 16, mais en remplaçant au stade A de l'exemple 16 l'acide 3-(2-imidazolyl) 4-aminobutyrique par l'acide 3-(2-(5-isopropyl benzofuryl)) 4-aminobutyrique, on obtient le composé du titre.

### EXEMPLE 20 : Ester méthylique de l'acide 3-(2-(5-isopropyl benzofuryl)) 4-methylamino butanoïque

Dissoudre 0,01 mole du composé de l'exemple 19 dans 100 cm³ de chloroforme. Ajouter goutte à goutte 0,011 mole de sulfate de diméthyle. Maintenir l'agitation pendant 3 heures et laver à l'eau puis sécher la phase organique. Evaporer à sec et purifier par chromatographie.

### EXEMPLE 21 : Acide 3-cyclopropylmethyl 4-amino butanoïque

En procédant comme dans l'exemple 1, mais en remplaçant au stade A de l'exemple 1 le 2-(5-isopropyl benzofuryl) carbaldéhyde par le cyclopropyl acétaldéhyde, on obtient
au

### STADE C : le 4-cyclopropylméthyl 2-oxopyrrolidine,

et au

### STADE D : le composé du titre.

### EXEMPLE 22 : Acide 3-(2-(4,5-dichloro imidazolyl)) 4-amino butanoïque

En procédant comme dans l'exemple 1, mais en remplaçant au stade A de l'exemple 1 le 2-(5-isopropyl benzofuryl) carbaldéhyde par le 2-(4,5-dichloro imidazolyl) carbaldéhyde, on obtient
au

### STADE C : le 4-(2-(4,5-dichloro imidazolyl)) 2-oxopyrrolidine,

et au

### STADE D : le composé du titre.

### EXEMPLE 23 : Acide 3-(2-(4-méthyl imidazolyl)) 4-amino butanoïque

En procédant comme dans l'exemple 1, mais en remplaçant au stade A de l'exemple 1 le 2-(5-isopropyl benzofuryl) carbaldéhyde par le 2-(4-méthyl imidazolyl) carbaldéhyde, on obtient
au

### STADE C : le 4-(2-(4-méthyl imidazolyl)) 2-oxopyrrolidine,

et au

### STADE D : le composé du titre

### EXEMPLE 24 : Acide 3-(2-(4,5-dichloro furyl)) 4-amino butanoïque

En procédant comme dans l'exemple 1, mais en remplaçant au stade A de l'exemple 1 le 2-(5-isopropyl benzofuryl) carbaldéhyde par le 2-(4,5-dichloro furyl) carbaldéhyde, on obtient
au

### STADE C : le 4-(2-(4,5-dichloro furyl)) 2-oxopyrrolidine,

et au

### STADE D : le composé du titre.

### EXEMPLE 25 : Acide 3-(2-(4-méthoxy benzothiényl)) 4-amino butanoïque

En procédant comme dans l'exemple 9, mais en utilisant initialement au stade A de l'exemple 9 le 2-(4-méthoxy benzothiényl) carbaldéhyde, on obtient
au

### STADE C : 4-(2-(4-méthoxy benzothiényl)) 2-oxopyrrolidine,

et au

### STADE D : 1-tert.butyloxycarbonyl 2-oxo 4-(2-(4-méthoxy benzothiényl)) pyrrolidine

et au

### STADE F : le composé du titre.

### EXEMPLE 26 : Ester méthylique de l'acide 3-(2-imidazolyl) 4-acétylamino butanoïque

Dissoudre 0,01 mole du composé obtenu au stade B de l'exemple 16 dans 50 cm³ de chloroforme. Ajouter 0,01 mole d'anhydride acétique et 0,015 mole de carbonate de sodium. Porter à reflux cinq heures sous agitation. Refroidir, évaporer à sec extraire au chloroforme à 2 reprises. Réunir les phases chloroformiques, sécher sur chlorure de calcium, et évaporer au bain-maire sous-vide. Purifier le résidu par chromatographie.

### EXEMPLE 27 : Acide 3-(2-(4-méthoxy furyl)) 4-amino butanoïque

En procédant comme dans l'exemple 1, mais en utilisant au stade A de l'exemple 1 le 2-(4-méthoxy furyl) carbaldéhyde au lieu du 2-(5-isopropyl benzofuryl) carbaldéhyde, on obtient
au

### STADE C : la 4-(2-(4-méthoxy furyl) 2-oxopyrrolidine,

et au

### STADE D : le composé du titre.

### EXEMPLE 28 : Acide 3-(4-pyridazinyl) 4-amino butanoïque

En procédant comme dans l'exemple 1, mais en utilisant au stade A de l'exemple 1 le 4-pyridazinyl carbaldéhyde au lieu du 2-(5-isopropyl benzofuryl) carbaldéhyde, on obtient
au

### STADE C : le 4-(4-pyridazinyl) 2-oxopyrrolidine,

et au

### STADE D : le composé du titre.

### EXEMPLE 29 : Acide 3-(2-pyrimidinyl) 4-amino butanoïque

En procédant comme dans l'exemple 1, mais en utilisant au stade A de l'exemple 1 le 2-pyrimidinyl carbaldéhyde au lieu du 2-(5-isopropyl benzofuryl) carbaldéhyde, on obtient
au

### STADE C : le 4-(2-pyrimidinyl) 2-oxopyrrolidine,

et au

### STADE D : le composé du titre.

### EXEMPLE 30 : Acide 3-(2-benzimidazolyl) 4-amino butanoïque

En procédant comme dans l'exemple 1, mais en utilisant au stade A de l'exemple 1 le 2-benzimidazolyl carbaldéhyde au lieu du 2-(5-isopropyl benzofuryl) carbaldéhyde, on obtient
au

### STADE C : la 2-(2-benzimidazolyl) 2-oxopyrrolidine,

et au

### STADE D : le composé du titre.

### EXEMPLE 31 : Acide 3-(3-azépinyl) 4-amino butanoïque

En procédant comme dans l'exemple 1, mais en utilisant au stade A de l'exemple 1, le 3-azépinyl carboxaldéhyde au lieu du 2-(5-isopropyl benzofuryl) carbaldéhyde, on obtient
au

### STADE C : la 4-(3-azépinyl) 2-oxopyrrolidine,

et au

### STADE D : le composé du titre.

### EXEMPLE 32 : Acide 3-(3-(1,4-diazépinyl)) 4-amino butanoïque

En procédant comme dans l'exemple 1, mais en utilisant au stade A de l'exemple 1 le 3-(1,4-diazépinyl) carboxaldéhyde au lieu du 2-(5-isopropyl benzofuryl) carbaldéhyde, on obtient
au

### STADE C : la 4-(3-(1,4-diazépinyl)) 2-oxopyrrolidine,

et au

### STADE D : le composé du titre.

### EXEMPLE 33 : Acide 3-(3-benzo[b]azépinyl) 4-amino butanoïque

En procédant comme dans l'exemple 1, mais en utilisant au stade A de l'exemple 1 le 3-benzo[b]azépinyl carbaldéhyde au lieu du 2-(5-isopropyl benzofuryl) carbaldéhyde, on obtient
au

### STADE C : la 4-(3-benzo[b]azépinyl) 2-oxopyrrolidine,

et au

### STADE D : le composé du titre.

### EXEMPLE 34 : Acide 3-(2-(1,3,5-triazinyl)) 4-amino butanoïque

En procédant comme dans l'exemple 1, mais en utilisant au stade A de l'exemple 1 le 2-(1,3,5-triazinyl) carbaldéhyde au lieu du 2-(5-isopropyl benzofuryl) carbaldéhyde, on obtient
au

### STADE C : la 4-(2-(1,3,5-triazinyl)) 2-oxopyrrolidine,

et au

### STADE D : le composé du titre.

### EXEMPLE 35 : Acide 3-(2-indolyl) 4-amino butanoïque

En procédant comme dans l'exemple 9, mais en utilisant initialement au stade A de l'exemple 9 le 2-indolyl carbaldéhyde, on obtient

### STADE C : 4-(2-indolyl) 2-oxopyrrolidine,

### STADE D : 1-tert.butyloxycarbonyl 2-oxo 4-(2-indolyl) pyrrolidine

et au

### STADE F : le composé du titre,

### EXEMPLE 36 : Acide 3-(2-(5-méthoxy indolyl)) 4-amino butanoïque

En procédant comme dans l'exemple 9, mais en utilisant initialement au stade A de l'exemple 9 le 2-(5-méthoxy indolyl) carbaldéhyde, on obtient

### STADE C : 4-(2-(5-méthoxy indolyl)) 2-oxopyrrolidine,

### STADE D : 1-tert.butyloxycarbonyl 2-oxo 4-(2-(5-méthoxy indolyl)) pyrrolidine

et au

### STADE F : le composé du titre.

### EXEMPLE 37 : Acide 3-(2-(5-méthyl indolyl)) 4-amino butanoïque

En procédant comme dans l'exemple 9, mais en utilisant initialement au stade A de l'exemple 9 le 2-(5-méthyl indolyl) carbaldéhyde, on obtient

### STADE C : 4-(2-(5-méthyl indolyl)) 2-oxopyrrolidine,

### STADE D : 1-tert.butyloxycarbonyl 2-oxo 4-(2-(5-méthyl indolyl)) pyrrolidine

et au

### STADE F : le composé du titre.

### EXEMPLE 38 : Acide 3-(2-(5-chloro indolyl)) 4-amino butanoïque

En procédant comme dans l'exemple 9, mais en utilisant initialement au stade A de l'exemple 9 le 2-(5-chloro indolyl) carbaldéhyde, on obtient

### STADE C : 4-(2-(5-chloro indolyl)) 2-oxopyrrolidine,

### STADE D : 1-tert.butyloxycarbonyl 2-oxo 4-(3-(5-chloro indolyl)) pyrrolidine

et au

### STADE F : le composé du titre,

### EXEMPLE 39 : Acide 4-tert.butyloxycarbonylamino 3-(2-(5-méthoxy benzofuryl)) butanoïque

En procédant comme dans l'exemple 9, mais en utilisant initialement au stade A de l'exemple 9 le 2-(5-méthoxy benzofuryl) carbaldéhyde, on obtient :
au

### STADE D : 1-tert.butyloxycarbonyl 2-oxo 4-(2-(5-méthoxy benzofuryl)) pyrrolidine

Point de fusion : 90 - 92 °C
Caractéristiques spectrales :
- infrarouge : 1710 - 1740 cm⁻¹ : ν CO
- RMN (CDCl₃) :
   δ : 1,53 ppm : singulet : (C(CH₃)₃)
   δ : 2,87 ppm : doublet : (CH₂CO)
   δ : 3,83 ppm : multiplet : (CH₃O, CH, CH₂ N)
   δ : 6,48 ppm : singulet : (H₃' ; benzofurane)
   δ : 6,87 ppm : doublet : (H₆' ; benzofurane)
   δ : 7,00 ppm : doublet : (H₄' ; benzofurane)
   δ : 7,44 ppm : doublet : (H₇' ; benzofurane)
et au

### STADE E : le produit du titre :

Point de fusion : 149 - 153 °C
Caractéristiques spectrales :
- infrarouge : 1700 - 1720 cm⁻¹ : ν CO
   3420 cm⁻¹ : ν NH
- RMN (CDCl₃) :
   δ : 1,40 ppm : singulet : (C(CH₃)₃)
   δ : 2,70 - 2,90 ppm : multiplet : (CH₂ CO)
   δ : 3,40 - 3,70 ppm : multiplet : (CH₂N, CH)
   δ : 3,82 ppm : singulet : (CH₃O)
   δ : 4,70 ppm : singulet : (NH)
   δ : 6,45 ppm : singulet : (H₃' ; benzofurane)
   δ : 6,82 ppm : doublet : (H₆' ; benzofurane)
   δ : 6,95 ppm : doublet : (H₄' ; benzofurane)
   δ : 7,33 ppm : doublet : (H₇' ; benzofurane)

### EXEMPLE 40 : 4-tert.butyloxycarbonylamino 3-(2-(5-méthoxy benzofuryl)) butanamide

### STADE A : chlorure de l'acide 4-tert.butyloxycarbonylamino 3-(2-(5-méthoxy benzofuryl)) butanamide

En procédant comme dans le stade A de l'exemple 16, mais en remplaçant l'acide 3-(2-imidazolyl) 4-aminobutyrique par le composé de l'exemple 39, on obtient le produit du stade A.

### STADE B : 4-tert.butyloxycarbonylamino 3-(2-(5-méthoxy benzofuryl)) butanamide

En procédant comme dans l'exemple 17, mais en remplaçant le chlorure de l'acide 3-(2-imidazolyl) 4-amino butanoïque par le composé obtenu au stade A de l'exemple 40, on obtient le produit du titre :
Point de fusion : 175 - 177 °C
Caractéristiques spectrales :
- infrarouge : 1665 - 1700 cm⁻¹ : νCO
   3400 cm⁻¹ : νNH

### ETUDE PHARMACOLOGIQUE DES COMPOSES DE L'INVENTION

### EXEMPLE 41 :

### ETUDE DE LA TOXICITE AIGUE

La toxicité aiguë a été appréciée après administration orale à des lots de 5 souris (20 ± 2 grammes) de doses croissantes (0,05 ; 0,1 ; 0,25 ; 0,50 ; 0,75 g/kg). Les animaux ont été observés à intervalles réguliers au cours de la première journée et quotidiennement pendant les deux semaines suivant le traitement.

Il apparaît que les composés de l'invention sont atoxiques.

### EXEMPLE 42 :

### ETUDE DE L'AFFINITE POUR LES RECEPTEURS GABA_{B}

Cette étude a été effectuée selon les techniques classiques d'étude de liaison aux récepteurs.

Il apparaît que les composés de l'invention ont une très forte affinité pour les récepteurs GABA_{B}.

Ainsi certains composés de l'invention ont une IC₅₀ de 0,05 »M en présence du R(-) [³H] baclofène. A titre de comparaison le meilleur composé de structure proche de l'art antérieur (J. Med. Chem. 1987, 30, 743-746) avait une IC₅₀ dans ce test de 0,61 »M et le baclofène une IC₅₀ de 0,33 »M.

### EXEMPLE 43 :

### ETUDE DE L'INHIBITION DE L'ACTIVITE EXCITATRICE INDUITE PAR DES AGENTS CONVULSIVANTS

Les composés de l'invention antagonisent l'activité excitatrice induite par des agents convulsivants dans des préparations d'hippocampe.

Des hippocampes ont été préparés à partir de cerveaux de rates adultes sacrifiées. Des coupes transversales ont été placées dans un milieu de conservation. Après 1 heure et 30 minutes d'incubation les préparations isolées ont été placées sous enregistrement, perfusées par une solution saline et oxygénées. Il apparaît des potentiels spontanés liés aux cellules pyramidales CA3.
On induit une excitation par addition de bicuculline (50»M).
On ajoute ensuite les composés de l'invention à diverses concentrations ou le baclofène.
Le baclofène ou les composés de l'invention sont perfusés pendant des périodes de 10 minutes. Le taux d'excitation est calculé pendant les quatre minutes précédant la perfusion du baclofène ou des composés de l'invention et durant les quatre dernières minutes de la perfusion des produits à étudier.
L'inhibition est exprimée en pourcentage d'excitation par rapport au taux initial, ce qui permet de terminer une concentration inhibitrice 50 (IC₅₀).
Les composés de l'invention ont une IC₅₀ comprise entre 5 et 20 »M, le baclofène dans ce test à une IC₅₀ voisine de 50 »M.

### EXEMPLE 44 :

### STIMULATION DE LA SYNTHESE D'AMP CYCLIQUE AU NIVEAU CEREBRAL

Les composés à tester sont administrés à la dose de 10 mg/kg par voie intrapéritonéale chez la souris de souche OF1 / IFFA Credo.
24 heures après la dernière injection, les animaux sont sacrifiés par congélation, l'AMPc présent dans ces structures cérébrales est dosé par radioimmunologie selon la méthode Amersham (protéine spécifique liante).
Certains composés de l'invention appraissent comme étant capables d'augmenter fortement la synthèse cérébrale d'AMP cyclique.

### EXEMPLE 45 :

### COMPOSITION PHARMACEUTIQUE : COMPRIMES

Comprimés dosés à 1,5 mg d'acide 3-(2-imidazolyl) 4-amino butanoïque.
Formule pour 1000 comprimés :

| | |
|---|---|
| Acide 3-(2-imidazolyl) 4-amino butanoïque | 1,5 g |
| Amidon de blé | 15 g |
| Amidon de maïs | 15 g |
| Lactose | 90 g |
| Stéarate de magnésium | 2 g |
| Silice | 1 g |
| Hydroxy propylcellulose | 2 g |

## Revendications (Revendications pour l'(les) Etat(s) contractant(s) suivant(s): AT, BE, CH, DE, DK, FR, GB, IT, LI, LU, NL, SE)

1. Composés de formule générale (I) : 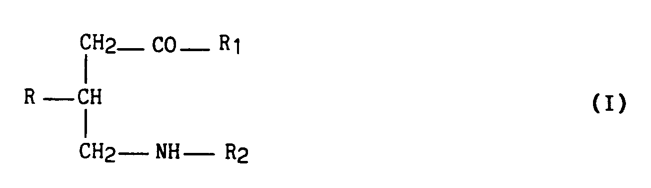 dans laquelle :
- R₁ représente un groupe hydroxy, amino, aminoalkyle inférieur ou alcoxy inférieur, ou un atome d'halogène,
- R₂ représente un atome d'hydrogène, un radical alkyle inférieur, un radical acyle inférieur, ou un radical alcoxycarbonyle inférieur,
- R représente :
. un radical de formule : 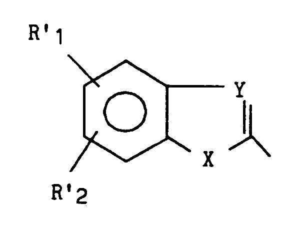 dans laquelle :
X représente un atome d'oxygène, de soufre, ou un groupement NH,
Y représente un atome de carbone, d'oxygène, ou d'azote,
R'₁ et R'₂ identiques ou différents, représentent un atome d'halogène ou d'hydrogène ou un radical alkyle inférieur ou alcoxy inférieur, hydroxy, nitro, amino, alkylamino inférieur ou trifluorométhyle,
avec la réserve que lorsque X est un atome d'oxygène, Y un atome de carbone, et R′₁ et R₂ chacun un atome d'hydrogène, alors R′₂ ne peut représenter ni un atome d'hydrogène ni un groupe méthoxy,
. un radical de formule : 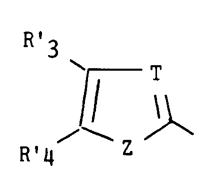 dans laquelle :
Z représente un atome d'oxygène, de soufre ou un groupement NH,
T représente un atome de carbone ou d'azote,
R′₃ et R′₄ identiques ou différents représentent un radical choisi parmi hydrogène, halogène, alkyle ou alcoxy inférieurs, hydroxy, nitro, amino, alkylamino inférieur, ou trifluorométhyle, avec la réserve que lorsque Z est un atome de soufre, T un atome de carbone et R′₃ un atome d'hydrogène, R′₄ ne peut pas être un groupement méthyle ou un atome d'hydrogène, de chlore ou de brome, et que lorsque Z est un atome d'oxygène, T un atome de carbone et R′₃ un atome d'hydrogène, R′₄ ne peut pas être un atome d'hydrogène ou un groupe méthyle,
. un radical cycloalkyle de 4 ou 5 atomes de carbone, cycloalkylalkyle ou dicycloalkylalkyle de 4 à 16 atomes de carbone éventuellement substitués sur le cycle par un groupement choisi parmi halogène, hydroxy, alkyle ou alcoxy inférieur, nitro, amino, alkylamino inférieur ou trifluorométhyle,
. un radical aromatique à 6 sommets éventuellement substitue et incluant dans son squelette carboné 2 ou 3 atomes d'azote,
. un radical aromatique à 6 sommets incluant dans son squelette carboné de 1 à 3 atomes d'azote et accolé à un cycle benzénique, chacun de ces 2 cycles pouvant être éventuellement substitué,
. ou un cycle à sept sommets, saturé ou non, incluant dans son squelette carboné un ou deux atomes d'azote éventuellement accolé à un cycle benzénique et éventuellement substitué sur le cycle azoté et/ou benzénique,
le terme substitué signifiant que les groupements qu'il affecte peuvent être substitués par un ou plusieurs atomes d'halogène ou groupements alkyle inférieur ou alcoxy inférieur, ou hydroxy, ou trifluorométhyle, ou nitro, ou amino, ou alkylamino inférieur,
leurs isomères optiques ainsi que le cas échéant leurs sels d'addition à une base ou un acide pharmaceutiquement acceptable,
étant entendu que, sauf précision contraire, les termes "alkyle inférieur", "alcoxycarbonyle inférieur", "alcoxy inférieur" "alkylamino inférieur" et "acyle inférieur" signifient des groupements contenant de 1 à 6 atomes de carbone, en chaine droite ou ramifiée.

2. Composés de formule (I) selon la revendication 1 dans laquelle R représente un radical de formule : 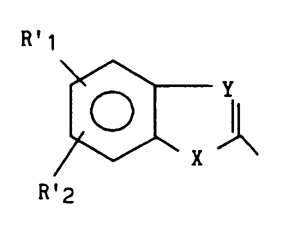 dans laquelle R'₁, R'₂, X et Y tels que définis dans la revendication 1, leurs isomères optiques ainsi que, le cas échéant, leurs sels d'addition à une base ou un acide pharmaceutiquement acceptable.

3. Composés de formule (I) selon la revendication 1 dans laquelle R représente un radical de formule : 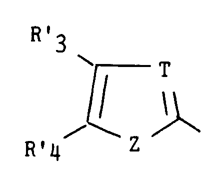 avec Z, T, R'₃, R'₄ tels que définis dans la revendication 1, leurs isomères optiques ainsi que le cas échéant leurs sels d'addition à un acide ou une base pharmaceutiquement acceptable.

4. Composés de formule (I) selon la revendication 1 pour lesquels R représente un cycloalkyle de 4 ou 5 atomes de carbone, cycloalkylalkyle ou dicycloalkylalkyle de 4 à 16 atomes de carbone, leurs isomères optiques ainsi que le cas échéant, leurs sels d'addition à une base ou un acide pharmaceutiquement acceptable.

5. Composés de formule (I) selon la revendication 1 dans lesquels R est un radical de formule : 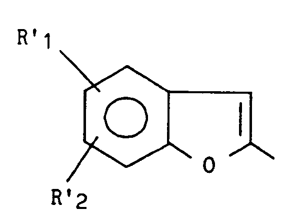 avec R'₁ et R'₂ identiques ou différents représentant un atome d'halogène ou d'hydrogène ou un radical alkyle inférieur, alcoxy inférieur ou trifluorométhyle avec la réserve que lorsque R'₁ et R₂ sont des atomes d'hydrogène, alors R'₂ ne peut représenter ni un atome d'hydrogène, ni un groupe méthoxy,
leurs isomères optiques ainsi que, le cas échéant, leurs sels d'addition à une base ou à un acide pharmaceutiquement acceptable.

6. Composés de formule (I) selon la revendication 1 dans lesquels R est un radical de formule : 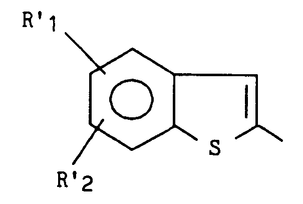 avec R'₁ et R'₂ identiques ou différents représentent un atome d'hydrogène ou d'halogène ou un radical alkyle inférieur, alcoxy inférieur ou trifluorométhyle, leurs isomères optiques ainsi que le cas échéant, leurs sels d'addition à une base ou un acide pharmaceutiquement acceptable.

7. Composés selon la revendication 1 pour lesquels R représente un groupement : 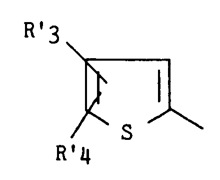 avec R'₃ et R'₄ identiques ou différents représentant un radical choisi parmi hydrogène, halogène, alkyle inférieur, alcoxy inférieur ou trifluorométhyle avec la réserve que si R'₃ est un atome d'hydrogène, alors R'₄ ne peut pas être un atome d'hydrogène, de chlore, ou de brome, ou un groupement méthyle,
leurs isomères optiques ainsi que le cas échéant, leurs sels d'addition à une base ou un acide pharmaceutiquement acceptable.

8. Composés selon la revendication 1 pour lesquels R représente un groupement : 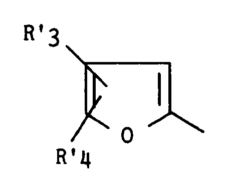 avec R'₃ et R'₄ identiques ou différents représentant un radical choisi parmi hydrogène, halogène, alkyle inférieur, alcoxy inférieur, ou trifluorométhyle avec la réserve que si R'₃ est un atome d'hydrogène, alors R'₄ ne peut pas être un atome d'hydrogène ou un groupement méthyle, leurs isomères optiques ainsi que le cas échéant, leurs sels d'addition à une base ou un acide pharmaceutiquement acceptable.

9. Composés selon la revendication 1 pour lesquels R représente un groupement : 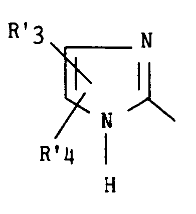 avec R'₃ et R'₄ identiques ou différents représentant un radical choisi parmi hydrogène, halogène, alkyle inférieur, alkoxy inférieur, trifluorométhyle,
leurs isomères optiques ainsi que, le cas échéant, leurs sels d'addition à une base ou un acide pharmaceutiquement acceptable.

10. Composés selon la revendication 1 pour lesquels R représente :
- un radical aromatique à 6 sommets éventuellement substitué et incluant dans son squelette carboné 2 ou 3 atomes d'azote,
- un radical aromatique à 6 sommets incluant dans son squelette carboné de 1 à 3 atomes d'azote et accolé à un cycle benzenique, chacun de ces 2 cycles pouvant être éventuellement substitué,
- ou un cycle à sept sommets, saturé ou non, incluant dans son squelette carboné 1 ou 2 atomes d'azote éventuellement accolé à un cycle benzénique et éventuellement substitué sur le cycle azoté et/ou benzénique,
le terme substitué signifiant que les groupements qu'il affecte peuvent être substitués par un ou plusieurs atomes d'halogène, ou groupements alkyle inférieur, ou alcoxy inférieur, ou hydroxy, ou trifluorométhyl, ou amino, ou alkylamino inférieur, ou nitro,
leurs isomères ainsi que leurs sels d'addition à un acide ou une base pharmaceutiquement acceptable.

11. Composés selon la revendication 1 pour lesquels R représente un radical indolyle, pyrimidinyle, pyridazinyle, 1,3,5-triazinyle, azépinyle éventuellement substitué par un groupement alkyle inférieur, alkoxy inférieur, trifluorométhyle ou par un ou plusieurs atomes d'halogène, leurs isomères ainsi que leurs sels d'addition à un acide ou à une base pharmaceutiquement acceptable.

12. Composé selon la revendication 1 qui est l'acide 3-(2-imidazolyl) 4-amino butanoïque, ses isomères ainsi que ses sels d'addition à un acide ou une base pharmaceutiquement acceptable.

13. Composé selon la revendication 1 qui est l'acide 3-(2-(5-isopropyl benzofuryl)) 4-amino butanoïque, ses isomères ainsi que ses sels d'addition à un acide ou une base pharmaceutiquement acceptable.

14. Composé selon la revendication 1 qui est l'acide 3-(2-(5-éthyl benzofuryl)) 4-amino butanoïque, ses isomères ainsi que ses sels d'addition à un acide ou une base pharmaceutiquement acceptable.

15. Composé selon la revendication 1 qui est l'acide 3-(2-(5-(1-methylpropyl) benzofuryl)) 4-amino butanoïque, ses isomères ainsi que ses sels d'addition à un acide ou une base pharmaceutiquement acceptable.

16. Composé selon la revendication 1 qui est l'acide 3-(2-(5-fluoro benzofuryl)) 4-amino butanoïque, ses isomères ainsi que ses sels d'addition à un acide ou une base pharmaceutiquement acceptable.

17. Composé selon la revendication 1 qui est l'acide 3-(2-(4,5-dichloro thiényl)) 4-amino butanoïque, ses isomères ainsi que ses sels d'addition à un acide ou une base pharmaceutiquement acceptable.

18. Composé selon la revendication 1 qui est l'acide 3-(2-(5-chloro benzofuryl)) 4-amino butanoïque, ses isomères ainsi que ses sels d'addition à un acide ou une base pharmaceutiquement acceptable.

19. Composé selon la revendication 1 qui est l'acide 3-(2-benzothiényl) 4-amino butanoïque, ses isomères ainsi que ses sels d'addition à un acide ou une base pharmaceutiquement acceptable.

20. Composé selon la revendication 1 qui est l'acide 3-(2-(5-methyl benzofuryl)) 4-amino butanoïque, ses isomères ainsi que ses sels d'addition à un acide ou une base pharmaceutiquement acceptable.

21. Composé selon la revendication 1 qui est l'acide 3-(2-(5-éthoxy benzofuryl)) 4-amino butanoïque, ses isomères ainsi que ses sels d'addition à un acide ou une base pharmaceutiquement acceptable.

22. Procédé de préparation des composés de formule (I) caractérisé en ce que l'on utilise comme matière première un compose de formule (II) : 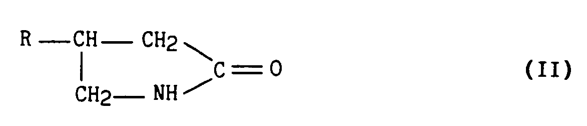 dans laquelle R a la même signification que dans la formule (I),
que l'on peut :
- soit hydrolyser par action d'un hydroxyde métallique pour obtenir après éventuelle purification un composé de formule (I/a) : 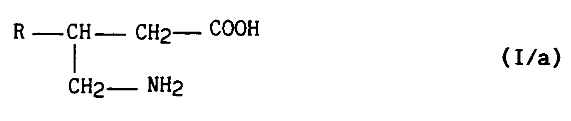 cas particulier des composés de formule I pour lesquels R₁ représente un groupement hydroxy, R₂ représente un atome d'hydrogène et R a la même signification que dans la formule (I),
- soit transformer par action d'un dicarbonate d'alkyle inférieur en présence d'une base forte en un composé de formule (III) : 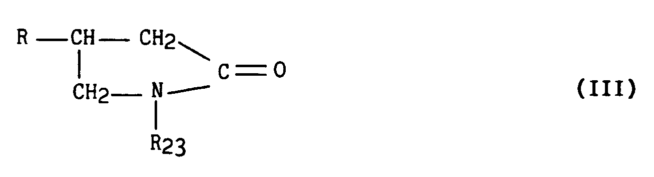 dans laquelle R a la même signification que précédemment et R₂₃ représente un groupement alcoxycarbonyle inférieur,
que l'on traite, après éventuelle purification, par un hydroxyde alcalin en milieu anhydre, puis par un acide pour obtenir un composé de formule (I/b) : 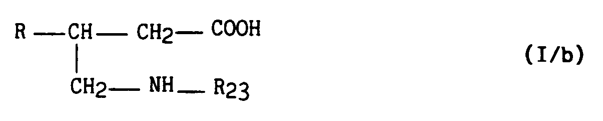 avec R et R₂₃ tels que définis précédemment,
cas particulier des composés de formule (I) pour lesquels R₁ représente un groupe hydroxy, R₂ représente un radical alcoxycarbonyle inférieur et R a la même signification que dans la formule (I),
qui traité en milieu acide, conduit à un composé de formule (I/a) tel que défini précédemment,
composé de formule (I/a) qui, quel que soit le procédé selon lequel il a été obtenu, peut être, si on le désire, transformé par un agent d'halogénation en son halogénure de formule (I/C) : 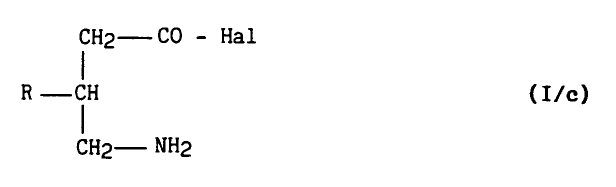 dans laquelle Hal représente un atome d'halogène et R a la même signification que dans la formule (I),
cas particulier des composés de formule (I) dans laquelle R₁ représente un atome d'halogène, R₂ représente un atome d'hydrogène, et R a la même signification que dans la formule (I),
composé de formule (I/a) ou (I/c) que l'on peut traiter, si on le désire :
- par un composé de formule :
**R₁'' - H**
dans laquelle R₁'' représente un groupement amino, alkylamino inférieur, ou alcoxy inférieur, pour conduire à un composé de formule (I) pour lequel R₁ représente un groupement amino, alkylamino inférieur ou alcoxy inférieur,
- et, si on le désire, par un agent d'alkylation comme le sulfate de diméthyle ou un halogénure d'alkyle de formule :
**R₂₁ - X**
dans laquelle R₂₁ signifie un groupement alkyle inférieur et X représente un atome d'halogène,
pour conduire à un composé de formule (I), dans lequel R₂ représente un groupement alkyle inférieur,
- ou bien encore, si on le désire, par un chlorure d'acide de formule :
**R₂₂Cl**
ou un anhydride d'acide de formule :
**R₂₂OR₂₂**
R₂₂ signifiant un groupement acyle inférieur,
pour conduire à un composé de formule (I) dans lequel R2 représente un groupement acyle inférieur,
les composés de formule (I) étant ensuite, si on le désire, soit dédoublés en leurs isomères optiques puis salifiés par addition d'une base ou d'un acide pharmaceutiquement acceptable, soit salifiés directement sous forme racémique par addition d'une base ou d'un acide pharmaceutiquement acceptable.

23. Composés de formule (II) : 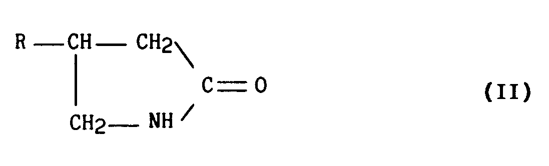 où R est tel que défini dans la revendication 1,
étant entendu que R ne peut représenter :
- un groupement 2-benzofuryle non substitué ou non substitué ou substitué sur le noyau benzénique par un atome de chlore, un atome de brome, ou un groupe méthoxy,
- ou un groupement 2-benzothiényle, utiles comme matières premières dans la synthèse des composés de l'invention de formule (I) selon la revendication 1, leurs isomères, ainsi que leurs éventuels sels d'addition à un acide ou à une base.

24. Composés de formule (III) : 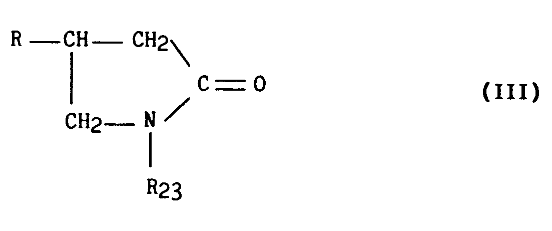 où R et R₂₃ sont tels que définis respectivement dans la revendication 1 et dans la revendication 22,
utiles comme matières premières dans la synthèse des composés de l'invention de formule (I) selon la revendication 1,
leurs isomères, ainsi que leurs éventuels sels d'addition à un acide ou à une base.

25. Composition pharmaceutique contenant comme principe actif au moins un composé selon l'une des revendications 1 à 21 en combinaison avec un ou plusieurs excipients ou véhicules inertes, non toxiques, pharmaceutiquement acceptables.

26. Composition pharmaceutique selon la revendication 25 contenant au moins 1 principe actif selon l'une des revendications 1 à 21 utilisable dans le traitement des troubles spastiques et des troubles de la sénescence.

## Revendications (Revendications pour l'(les) Etat(s) contractant(s) suivant(s): ES, GR)

1. Procédé de préparation des composés de formule générale (I) : 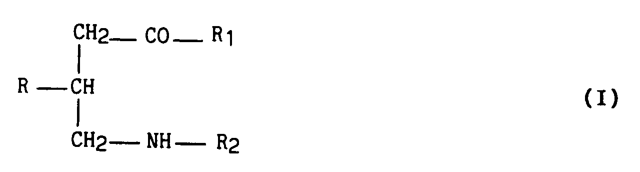 dans laquelle :
- R₁ représente un groupe hydroxy, amino, aminoalkyle inférieur ou alcoxy inférieur, ou un atome d'halogène,
- R₂ représente un atome d'hydrogène, un radical alkyle inférieur, un radical acyle inférieur, ou un radical alcoxycarbonyle inférieur,
- R représente :
. un radical de formule : 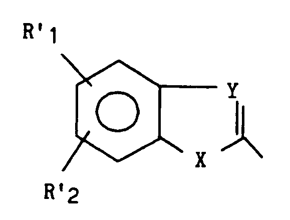 dans laquelle :
X représente un atome d'oxygène, de soufre, ou un groupement NH,
Y représente un atome de carbone, d'oxygène, ou d'azote,
R'₁ et R'₂ identiques ou différents, représentent un atome d'halogène ou d'hydrogène ou un radical alkyle inférieur ou alcoxy inférieur, hydroxy, nitro, amino, alkylamino inférieur ou trifluorométhyle,
avec la réserve que lorsque X est un atome d'oxygène, Y un atome de carbone, et R'₁ et R₂ chacun un atome d'hydrogène, alors R'2 ne peut représenter ni un atome d'hydrogène ni un groupe méthoxy,
. un radical de formule : 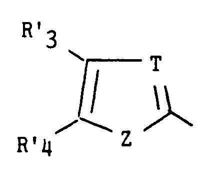 dans laquelle :
Z représente un atome d'oxygène, de soufre ou un groupement NH,
T représente un atome de carbone ou d'azote,
R'₃ et R'₄ identiques ou différents représentent un radical choisi parmi hydrogène, halogène, alkyle ou alcoxy inférieurs, hydroxy, nitro, amino, alkylamino inférieur, ou trifluorométhyle, avec la réserve que lorsque Z est un atome de soufre, T un atome de carbone et R'₃ un atome d'hydrogène, R'₄ ne peut pas être un groupement méthyle ou un atome d'hydrogène, de chlore ou de brome, et que lorsque Z est un atome d'oxygène, T un atome de carbone et R'₃ un atome d'hydrogène, R'₄ ne peut pas être un atome d'hydrogène ou un groupe méthyle,
. un radical cycloalkyle de 4 ou 5 atomes de carbone, cycloalkylalkyle ou dicycloalkylalkyle de 4 à 16 atomes de carbone éventuellement substitués sur le cycle par un groupement choisi parmi halogène, hydroxy, alkyle ou alcoxy inférieur, nitro, amino, alkylamino inférieur ou trifluorométhyle,
. un radical aromatique à 6 sommets éventuellement substitué et incluant dans son squelette carboné 2 ou 3 atomes d'azote,
. un radical aromatique à 6 sommets incluant dans son squelette carboné de 1 à 3 atomes d'azote et accolé à un cycle benzénique, chacun de ces 2 cycles pouvant être éventuellement substitué,
. ou un cycle à sept sommets, saturé ou non, incluant dans son squelette carboné un ou deux atomes d'azote éventuellement accolé à un cycle benzénique et éventuellement substitué sur le cycle azoté et/ou benzénique,
le terme substitué signifiant que les groupements qu'il affecte peuvent être substitués par un ou plusieurs atomes d'halogène ou groupements alkyle inférieur ou alcoxy inférieur, ou hydroxy, ou trifluorométhyle, ou nitro, ou amino, ou alkylamino inférieur,
de leurs isomères optiques ainsi que, le cas échéant, de leurs sels d'addition à une base ou un acide pharmaceutiquement acceptable,
étant entendu que, sauf précision contraire, les termes "alkyle inférieur", "alcoxycarbonyle inférieur", "alcoxy inférieur" "alkylamino inférieur" et "acyle inférieur" signifient des groupements contenant de 1 à 6 atomes de carbone, en chaine droite ou ramifiée,
caractérisé en ce que,
on utilise comme matière première un composé de formule (II) : 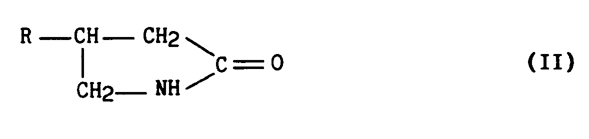 dans laquelle R a la même signification que dans la formule (I),
que l'on peut :
- soit hydrolyser par action d'un hydroxyde métallique pour obtenir après éventuelle purification un composé de formule (I/a) : 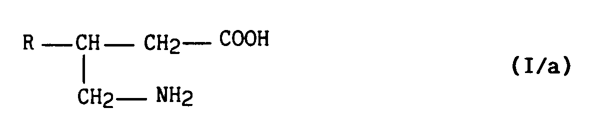 cas particulier des composés de formule I pour lesquels R₁ représente un groupement hydroxy, R₂ représente un atome d'hydrogène et R a la même signification que dans la formule (I),
- soit transformer par action d'un dicarbonate d'alkyle inférieur en présence d'une base forte en un composé de formule (III) : 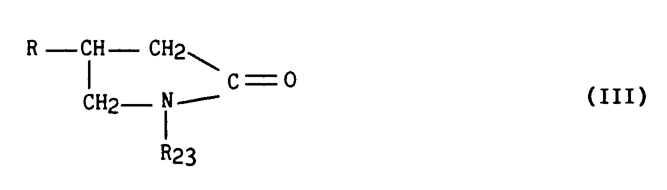 dans laquelle R a la même signification que précédemment et R₂₃ représente un groupement alcoxycarbonyle inférieur,
que l'on traite, après éventuelle purification, par un hydroxyde alcalin en milieu anhydre, puis par un acide pour obtenir un composé de formule (I/b) : 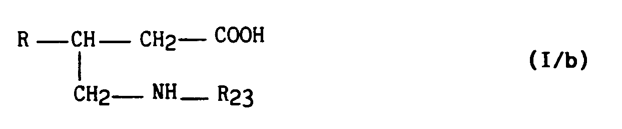 avec R et R₂₃ tels que définis précédemment,
cas particulier des composés de formule (I) pour lesquels R₁ représente un groupe hydroxy, R₂ représente un radical alcoxycarbonyle inférieur et R a la même signification que dans la formule (I),
qui traité en milieu acide, conduit à un composé de formule (I/a) tel que défini précédemment,
composé de formule (I/a) qui, quel que soit le procédé selon lequel il a été obtenu, peut être, si on le désire, transformé par un agent d'halogénation en son halogénure de formule (I/C) : 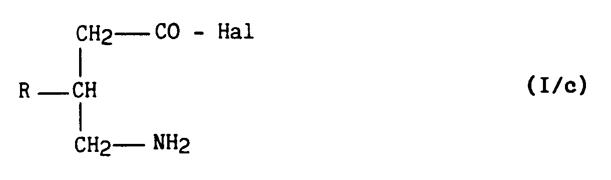 dans laquelle Hal représente un atome d'halogène et R a la même signification que dans la formule (I),
cas particulier des composés de formule (I) dans laquelle R₁ représente un atome d'halogène, R₂ représente un atome d'hydrogène, et R a la même signification que dans la formule (I),
composé de formule (I/a) ou (I/c) que l'on peut traiter, si on le désire :
- par un composé de formule :
**R₁'' - H**
dans laquelle R₁'' représente un groupement amino, alkylamino inférieur, ou alcoxy inférieur, pour conduire à un composé de formule (I) pour lequel R₁ représente un groupement amino, alkylamino inférieur ou alcoxy inférieur,
- et, si on le désire, par un agent d'alkylation comme le sulfate de diméthyle ou un halogénure d'alkyle de formule :
**R₂₁ - X**
dans laquelle R₂₁ signifie un groupement alkyle inférieur et X représente un atome d'halogène,
pour conduire à un composé de formule (I), dans lequel R₂ représente un groupement alkyle inférieur,
- ou bien encore, si on le désire, par un chlorure d'acide de formule :
**R₂₂Cl**
ou un anhydride d'acide de formule :
**R₂₂OR₂₂**
R₂₂ signifiant un groupement acyle inférieur,
pour conduire à un composé de formule (I) dans lequel R2 représente un groupement acyle inférieur,
les composés de formule (I) étant ensuite, si on le désire, soit dédoublés en leurs isomères optiques puis salifiés par addition d'une base ou d'un acide pharmaceutiquement acceptable, soit salifiés directement sous forme racémique par addition d'une base ou d'un acide pharmaceutiquement acceptable.

2. Procédé de préparation selon la revendication 1 des composés de formule (I) dans laquelle R représente un radical de formule : 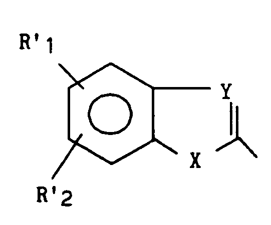 dans laquelle R'₁, R'₂, X et Y tels que définis dans la revendication 1, de leurs isomères optiques ainsi que, le cas échéant, de leurs sels d'addition à une base ou un acide pharmaceutiquement acceptable.

3. Procédé de préparation selon la revendication 1 des composés de formule (I) dans laquelle R représente un radical de formule : 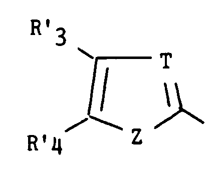 avec Z, T, R'₃, R'₄ tels que définis dans la revendication 1, de leurs isomères optiques ainsi que, le cas échéant, de leurs sels d'addition à un acide ou une base pharmaceutiquement acceptable.

4. Procédé de préparation selon la revendication 1 des composés de formule (I) pour lesquels R représente un cycloalkyle de 4 ou 5 atomes de carbone, cycloalkylalkyle ou dicycloalkylalkyle de 4 à 16 atomes de carbone, de leurs isomères optiques ainsi que, le cas échéant, de leurs sels d'addition à une base ou un acide pharmaceutiquement acceptable.

5. Procédé de préparation selon la revendication 1 des composés de formule (I) dans lesquels R est un radical de formule : 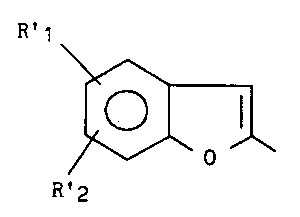 avec R'₁ et R'₂ identiques ou différents représentant un atome d'halogène ou d'hydrogène ou un radical alkyle inférieur, alcoxy inférieur ou trifluorométhyle avec la réserve que lorsque R'₁ et R₂ sont des atomes d'hydrogène, alors R'₂ ne peut représenter ni un atome d'hydrogène, ni un groupe méthoxy,
de leurs isomères optiques ainsi que, le cas échéant, de leurs sels d'addition à une base ou à un acide pharmaceutiquement acceptable.

6. Procédé de préparation selon la revendication 1 des composés de formule (I) dans lesquels R est un radical de formule : 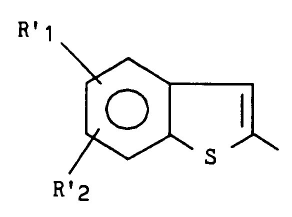 avec R'₁ et R'₂ identiques ou différents représentent un atome d'hydrogène ou d'halogène ou un radical alkyle inférieur, alcoxy inférieur ou trifluorométhyle, de leurs isomères optiques ainsi que, le cas échéant, de leurs sels d'addition à une base ou un acide pharmaceutiquement acceptable.

7. Procédé de préparation selon la revendication 1 des composés de formule (I) pour lesquels R représente un groupement : 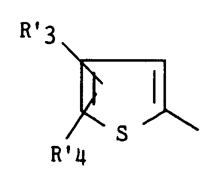 avec R'₃ et R'₄ identiques ou différents représentant un radical choisi parmi hydrogène, halogène, alkyle inférieur, alcoxy inférieur ou trifluorométhyle avec la réserve que si R'₃ est un atome d'hydrogène, alors R'₄ ne peut pas être un atome d'hydrogène, de chlore, ou de brome, ou un groupement méthyle,
de leurs isomères optiques ainsi que, le cas échéant, de leurs sels d'addition à une base ou un acide pharmaceutiquement acceptable.

8. Procédé de préparation selon la revendication 1 des composés de formule (I) pour lesquels R représente un groupement : 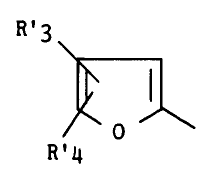 avec R'₃ et R'₄ identiques ou différents représentant un radical choisi parmi hydrogène, halogène, alkyle inférieur, alcoxy inférieur, ou trifluorométhyle avec la réserve que si R'₃ est un atome d'hydrogène, alors R'₄ ne peut pas être un atome d'hydrogène ou un groupement méthyle, de leurs isomères optiques ainsi que, le cas échéant, de leurs sels d'addition à une base ou un acide pharmaceutiquement acceptable.

9. Procédé de préparation selon la revendication 1 des composés de formule (I) pour lesquels R représente un groupement : 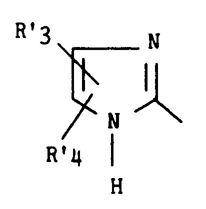 avec R'₃ et R'₄ identiques ou différents représentant un radical choisi parmi hydrogène, halogène, alkyle inférieur, alkoxy inférieur, trifluorométhyle,
de leurs isomères optiques ainsi que, le cas échéant, de leurs sels d'addition à une base ou un acide pharmaceutiquement acceptable.

10. Procédé de préparation selon la revendication 1 des composés de formule (I) pour lesquels R représente :
- un radical aromatique à 6 sommets éventuellement substitué et incluant dans son squelette carboné 2 ou 3 atomes d'azote,
- un radical aromatique à 6 sommets incluant dans son squelette carboné de 1 à 3 atomes d'azote et accolé à un cycle benzenique, chacun de ces 2 cycles pouvant être éventuellement substitué,
- ou un cycle à sept sommets, saturé ou non, incluant dans son squelette carboné 1 ou 2 atomes d'azote éventuellement accolé à un cycle benzénique et éventuellement substitué sur le cycle asoté et/ou benzénique,
le terme substitué signifiant que les groupements qu'il affecte peuvent être substitués par un ou plusieurs atomes d'halogène, ou groupements alkyle inférieur, ou alcoxy inférieur, ou hydroxy, ou trifluorométhyl, ou amino, ou alkylamino inférieur, ou nitro,
de leurs isomères ainsi que, le cas échéant, de leurs sels d'addition à un acide ou une base pharmaceutiquement acceptable.

11. Procédé de préparation selon la revendication 1 des composés de formule (I) pour lesquels R représente un radical indolyle, pyrimidinyle, pyridazinyle, 1,3,5-triazinyle, azépinyle éventuellement substitué par un groupement alkyle inférieur, alkoxy inférieur, trifluorométhyle ou par un ou plusieurs atomes d'halogène, de leurs isomères ainsi que, le cas échénat, de leurs sels d'addition à un acide ou à une base pharmaceutiquement acceptable.

12. Procédé de préparation selon la revendication 1 du composé qui est l'acide 3-(2-imidazolyl) 4-amino butanoïque, de ses isomères ainsi que de ses sels d'addition à un acide ou une base pharmaceutiquement acceptable.

13. Procédé de préparation selon la revendication 1 du composé qui est l'acide 3-(2-(5-isopropyl benzofuryl)) 4-amino butanoïque, de ses isomères ainsi que de ses sels d'addition à un acide ou une base pharmaceutiquement acceptable.

14. Procédé de préparation selon la revendication 1 du composé qui est l'acide 3-(2-(5-éthyl benzofuryl)) 4-amino butanoïque, de ses isomères ainsi que de ses sels d'addition à un acide ou une base pharmaceutiquement acceptable.

15. Procédé de préparation selon la revendication 1 du composé qui est l'acide 3-(2-(5-(1-méthylpropyl) benzofuryl)) 4-amino butanoïque, de ses isomères ainsi que de ses sels d'addition à un acide ou une base pharmaceutiquement acceptable.

16. Procédé de préparation selon la revendication 1 du composé qui est l'acide 3-(2-(5-fluoro benzofuryl)) 4-amino butanoïque, de ses isomères ainsi que de ses sels d'addition à un acide ou une base pharmaceutiquement acceptable.

17. Procédé de préparation selon la revendication 1 du composé qui est l'acide 3-(2-(4,5-dichloro thiényl)) 4-amino butanoïque, de ses isomères ainsi que de ses sels d'addition à un acide ou une base pharmaceutiquement acceptable.

18. Procédé de préparation selon la revendication 1 du composé qui est l'acide 3-(2-(5-chloro benzofuryl)) 4-amino butanoïque, de ses isomères ainsi que de ses sels d'addition à un acide ou une base pharmaceutiquement acceptable.

19. Procédé de préparation selon la revendication 1 du composé qui est l'acide 3-(2-benzothiényl) 4-amino butanoïque, de ses isomères ainsi que de ses sels d'addition à un acide ou une base pharmaceutiquement acceptable.

20. Procédé de préparation selon la revendication 1 du composé qui est l'acide 3-(2-(5-méthyl benzofuryl)) 4-amino butanoïque, de ses isomères ainsi que de ses sels d'addition à un acide ou une base pharmaceutiquement acceptable.

21. Procédé de préparation selon la revendication 1 du composé qui est l'acide 3-(2-(5-éthoxy benzofuryl)) 4-amino butanoïque, de ses isomères ainsi que de ses sels d'addition à un acide ou une base pharmaceutiquement acceptable.

22. Procédé de préparation des composés de formule (II) : 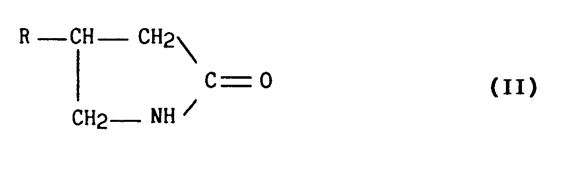 où R est tel que défini dans la revendication 1,
étant entendu que R ne peut représenter :
- un groupement 2-benzofuryle non substitué ou substitué sur le noyau benzénique par un atome de chlore, un atome de brome, ou un groupe méthoxy,
- ou un groupement 2-benzothiényle,
de leurs isomères, ainsi que de leurs éventuels sels d'addition à un acide ou à une base,
caractérisé en ce que,
- soit on condense dans un solvant apolaire aprotique un aldéhyde de formule (V):
**R - CHO (V)**
dans laquelle R a la même signification précédemment,
avec un ester de carboxyméthylidène triphénylphosphorane de formule (VI) :
**(C₆H₅)₃ - P = CH - COOR' (VI)**
dans laquelle R' représente un radical alkyle inférieur,
pour obtenir un ester de formule (VII) :
**R - CH = CH - COOR' (VII)**
dans laquelle R et R' ont les mêmes significations que précédemment,
que l'on condense, en milieu polaire protique avec le nitrométhane en présence d'une base forte, pour obtenir un composé de formule (VIII) : 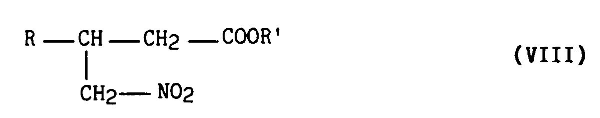 dans laquelle R et R' ont les mêmes significations que précédemment,
qui est réduit en milieu alcoolique par action de l'hydrogène en présence d'un catalyseur métallique, en un composé de formule (IX) : 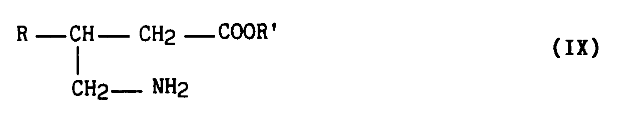 dans laquelle R et R' ont les mêmes significations que précédemment,
qui est cyclisé par chauffage en un composé de formule (II) : 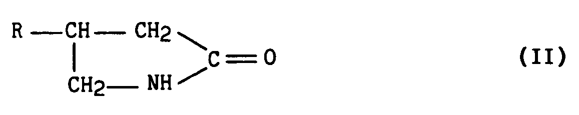 dans laquelle R a la même signification que précédemment,
- soit on traite d'un composé de formule (X) :
**RCOCH₃ (X)**
dans laquelle R a la même définition que précédemment,
à chaud en présence de zinc par un composé de formule (XI) :
**Br - CH₂ - COOA (XI)**
dans laquelle A représente un groupement alkyle inférieur,
pour conduire, après hydrolyse acide, extraction et purification éventuelles, à un composé de formule (XII) : 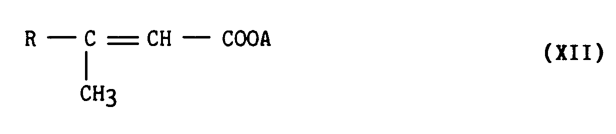 dans laquelle R et A ont la même signification que précédemment,
que l'on traite par la N bromo succinimide pour conduire à un composé de formule (XIII) : 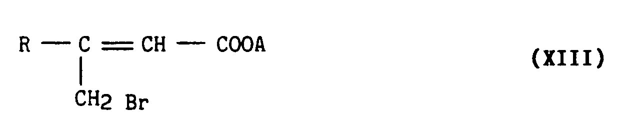 dans laquelle R et A ont la même signification que précédemment,
que l'on traite par l'ammoniac préférentiellement en excès pour conduire à un composé de formule (XIV) : 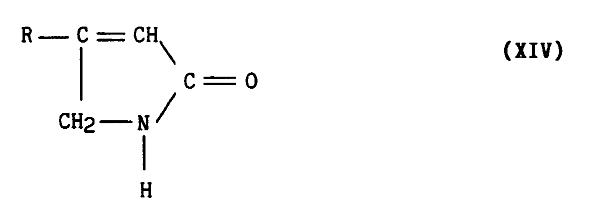 dans lequel R a la même définition que précédemment,
que l'on soumet à une hydrogénation catalytique pour obtenir un composé de formule (II) tel que ci-dessus défini.

23. Procédé de préparation des compositions pharmaceutiques contenant comme principe actif au moins un composé préparé selon l'une des revendications 1 à 21 en combinaison avec un ou plusieurs excipients ou véhicules inertes, non toxiques, pharmaceutiquement acceptables.

24. Procédé de préparation des compositions pharmaceutiques selon la revendication 23 contenant au moins 1 principe actif préparé selon l'une des revendications 1 à 21 utilisable dans le traitement des troubles spastiques et des troubles de la sénescence.

## Claims (Claims for the following Contracting State(s): AT, BE, CH, DE, DK, FR, GB, IT, LI, LU, NL, SE)

1. Compounds of the general formula (I): 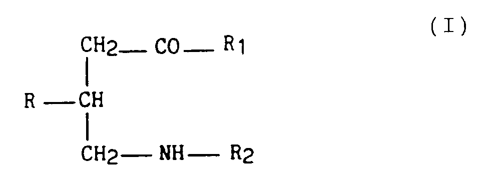 in which:
- R₁ represents a hydroxy, amino, lower alkylamino or lower alkoxy group, or a halogen atom,
- R₂ represents a hydrogen atom, a lower alkyl radical, a lower acyl radical, or a lower alkoxycarbonyl radical,
- R represents:
. a radical of the formula 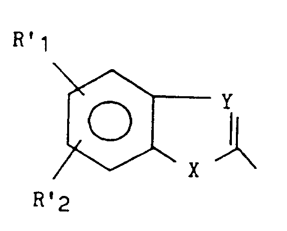 in which:
X represents an oxygen or sulphur atom or an NH grouping,
Y represents a carbon, oxygen or nitrogen atom,
R'₁ and R'₂, which are identical or different, represent a halogen or hydrogen atom or a lower alkyl, lower alkoxy, hydroxy, nitro, amino, lower alkylamino or trifluoromethyl radical,
with the proviso that, when X is an oxygen atom, Y is a carbon atom and each of R'₁ and R₂ is a hydrogen atom, then R'₂ can represent neither a hydrogen atom nor a methoxy group,
. a radical of the formula 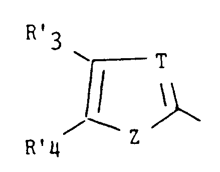 in which:
Z represents an oxygen or sulphur atom or an NH grouping,
T represents a carbon or nitrogen atom,
R'₃ and R'₄, which are identical or different, represent a radical selected from hydrogen, halogen, lower alkyl, lower alkoxy, hydroxy, nitro, amino, lower alkylamino or trifluoromethyl, with the proviso that, when Z is a sulphur atom, T is a carbon atom and R'₃ is a hydrogen atom, R'₄ may not be a methyl grouping or a hydrogen, chlorine or bromine atom, and that, when Z is an oxygen atom, T is a carbon atom and R'₃ is a hydrogen atom, R'₄ may not be a hydrogen atom or a methyl group,
. a cycloalkyl radical having 4 or 5 carbon atoms or a cycloalkylalkyl or dicycloalkylalkyl radical having from 4 to 16 carbon atoms, optionally substituted on the ring by a grouping selected from halogen, hydroxy, lower alkyl, lower alkoxy, nitro, amino, lower alkylamino or trifluoromethyl,
. an optionally substituted aromatic radical having 6 ring members that includes 2 or 3 nitrogen atoms in its carbon skeleton,
. an aromatic radical having 6 ring members that includes from 1 to 3 nitrogen atoms in its carbon skeleton and is fused to a benzene ring, it being optionally possible for each one of these 2 rings to be substituted,
. or a saturated or unsaturated ring having seven ring members that includes one or two nitrogen atoms in its carbon skeleton and is optionally fused to a benzene ring and optionally is substituted on the nitrogen and/or benzene ring,
the term "substituted" indicating that the groupings so qualified can be substituted by one or more halogen atoms or lower alkyl or lower alkoxy, or hydroxy, or trifluoromethyl, or nitro, or amino, or lower alkylamino groupings,
their optical isomers and also, where appropriate, their salts of addition with a pharmaceutically acceptable base or acid,
it being understood that, unless otherwise indicated, the terms "lower alkyl", "lower alkoxycarbonyl", "lower alkoxy", "lower alkylamino" and "lower acyl" indicate groupings containing from 1 to 6 carbon atoms in a straight or branched chain.

2. Compounds of formula (I) according to claim 1 in which R represents a radical of the formula: 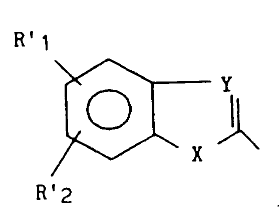 in which R'₁, R'₂, X and Y are as defined in claim 1, their optical isomers and also, where appropriate, their salts of addition with a pharmaceutically acceptable base or acid.

3. Compounds of formula (I) according to claim 1, in which R represents a radical of the formula: 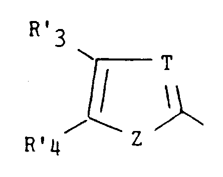 in which Z, T, R'₃ and R'₄ are as defined in claim 1, their optical isomers and also, where appropriate, their salts of addition with a pharmaceutically acceptable acid or base.

4. Compounds of formula (I) according to claim 1, in which R represents a cycloalkyl radical having 4 or 5 carbon atoms, a cycloalkylalkyl or dicycloalkylalkyl radical having from 4 to 16 carbon atoms, their optical isomers and also, where appropriate, their salts of addition with a pharmaceutically acceptable base or acid.

5. Compounds of formula (I) according to claim 1, in which R is a radical of the formula: 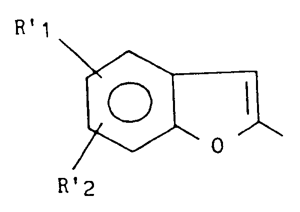 in which R'₁ and R'₂, which are identical or different, represent a halogen or hydrogen atom or a lower alkyl, lower alkoxy or trifluoromethyl radical, with the proviso that, when R'₁ and R₂ are hydrogen atoms, R'₂ can represent neither a hydrogen atom nor a methoxy group, their optical isomers and also, where appropriate, their salts of addition with a pharmaceutically acceptable base or acid.

6. Compounds of formula (I) according to claim 1, in which R is a radical of the formula: 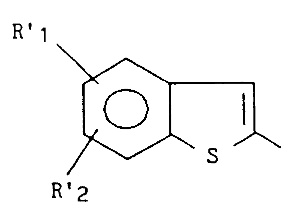 in which R'₁ and R'₂, which are identical or different, represent a hydrogen or halogen atom or a lower alkyl, lower alkoxy or trifluoromethyl radical, their optical isomers and also, where appropriate, their salts of addition with a pharmaceutically acceptable base or acid.

7. Compounds according to claim 1, in which R represents a grouping: 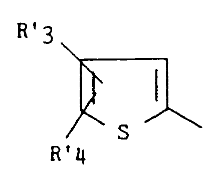 in which R'₃ and R'₄, which are identical or different, represent a radical selected from hydrogen, halogen, lower alkyl, lower alkoxy or trifluoromethyl, with the proviso that, if R'₃ is a hydrogen atom, then R'₄ may not be a hydrogen, chlorine or bromine atom or a methyl grouping,
their optical isomers and also, where appropriate, their salts of addition with a pharmaceutically acceptable base or acid.

8. Compounds according to claim 1, in which R represents a grouping: 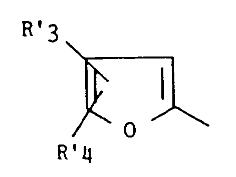 in which R'₃ and R'₄, which are identical or different, represent a radical selected from hydrogen, halogen, lower alkyl, lower alkoxy or trifluoromethyl, with the proviso that, if R'₃ is a hydrogen atom, then R'₄ may not be a hydrogen atom or a methyl grouping,
their optical isomers and also, where appropriate, their salts of addition with a pharmaceutically acceptable base or acid.

9. Compounds according to claim 1, in which R represents a grouping: 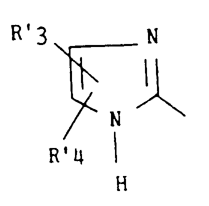 in which R'₃ and R'₄, which are identical or different, represent a radical selected from hydrogen, halogen, lower alkyl, lower alkoxy or trifluoromethyl, their optical isomers and also, where appropriate, their salts of addition with a pharmaceutically acceptable base or acid.

10. Compounds according to claim 1 in which R represents:
- an optionally substituted aromatic radical having 6 ring members that includes 2 or 3 nitrogen atoms in its carbon skeleton,
- an aromatic radical having 6 ring members that includes from 1 to 3 nitrogen atoms in its carbon skeleton and is fused to a benzene ring, it being optionally possible for each one of these 2 rings to be substituted,
- or a saturated or unsaturated ring having seven ring members that includes 1 or 2 nitrogen atoms in its carbon skeleton and is optionally fused to a benzene ring and optionally is substituted on the nitrogen and/or benzene ring,
the term "substituted" indicating that the groupings so qualified can be substituted by one or more halogen atoms or lower alkyl or lower alkoxy or hydroxy or trifluoromethyl or amino or lower alkylamino or nitro groupings,
their isomers and also their salts of addition with a pharmaceutically acceptable acid or base.

11. Compounds according to claim 1, in which R represents an indolyl, pyrimidinyl, pyridazinyl, 1,3,5-triazinyl or azepinyl radical optionally substituted by a lower alkyl, lower alkoxy or trifluoromethyl grouping or by one or more halogen atoms, their isomers and also their salts of addition with a pharmaceutically acceptable acid or base.

12. Compound according to claim 1 which is 3-(2-imidazolyl)-4-aminobutanoic acid, its isomers and also its salts of addition with a pharmaceutically acceptable acid or base.

13. Compound according to claim 1 which is 3-(2-(5-isopropylbenzofuryl))-4-aminobutanoic acid, its isomers and also its salts of addition with a pharmaceutically acceptable acid or base.

14. Compound according to claim 1 which is 3-(2-(5-ethylbenzofuryl))-4-aminobutanoic acid, its isomers and also its salts of addition with a pharmaceutically acceptable acid or base.

15. Compound according to claim 1 which is 3-(2-(5-(1-methylpropyl)benzofuryl))-4-aminobutanoic acid, its isomers and also its salts of addition with a pharmaceutically acceptable acid or base.

16. Compound according to claim 1 which is 3-(2-(5-fluorobenzofuryl))-4-aminobutanoic acid, its isomers and also its salts of addition with a pharmaceutically acceptable acid or base.

17. Compound according to claim 1 which is 3-(2-(4,5-dichlorothienyl))-4-aminobutanoic acid, its isomers and also its salts of addition with a pharmaceutically acceptable acid or base.

18. Compound according to claim 1 which is 3-(2-(5-chlorobenzofuryl))-4-aminobutanoic acid, its isomers and also its salts of addition with a pharmaceutically acceptable acid or base.

19. Compound according to claim 1 which is 3-(2-benzothienyl)-4-aminobutanoic acid, its isomers and also its salts of addition with a pharmaceutically acceptable acid or base.

20. Compound according to claim 1 which is 3-(2-(5-methylbenzofuryl))-4-aminobutanoic acid, its isomers and also its salts of addition with a pharmaceutically acceptable acid or base.

21. Compound according to claim 1 which is 3-(2-(5-ethoxybenzofuryl))-4-aminobutanoic acid, its isomers and also its salts of addition with a pharmaceutically acceptable acid or base.

22. Process for the preparation of the compounds of formula (I), characterised in that there is used as starting material a compound of formula (II): 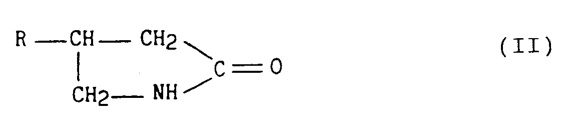 in which R is as defined in formula (I),
which can be:
- either hydrolysed by the action of a metal hydroxide to obtain, after optional purification, a compound of formula (I/a): 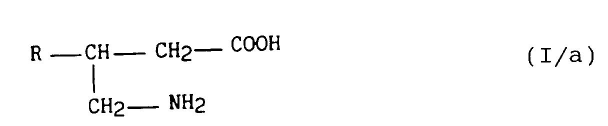
a particular case of the compounds of formula (I) in which R₁ represents a hydroxy grouping, R₂ represents a hydrogen atom and R is as defined in formula (I),
- or converted by the action of a lower alkyl dicarbonate in the presence of a strong base into a compound of formula (III): 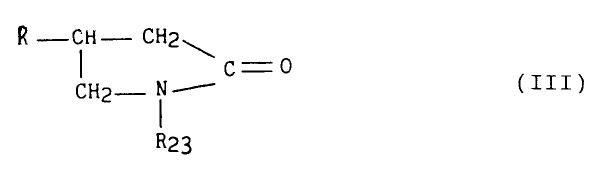
in which R is as defined above and R₂₃ represents a lower alkoxycarbonyl grouping,
which is treated, after optional purification, with an alkali metal hydroxide in an anhydrous medium, and then with an acid to obtain a compound of formula (I/b): 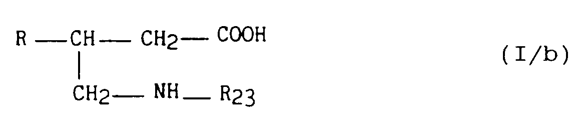 in which R and R₂₃ are as defined above,
a particular case of the compounds of formula (I) in which R₁ represents a hydroxy group, R₂ represents a lower alkoxycarbonyl radical and R is as defined in formula (I),
which, treated in an acidic medium, yields a compound of formula (I/a) as defined above,
which compound of formula (I/a), irrespective of the process by which it has been obtained, may, if desired, be converted by a halogenating agent into its halide of formula (I/c): 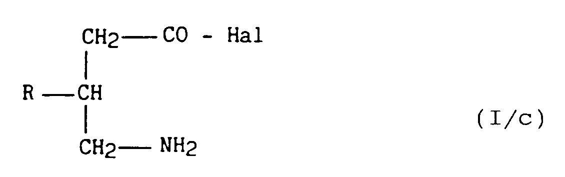 in which Hal represents a halogen atom and R is as defined in formula (I),
a particular case of the compounds of formula (I) in which R₁ represents a halogen atom, R₂ represents a hydrogen atom and R is as defined in formula (I),
which compound of formula (I/a) or (I/c) can be treated, if desired:
- with a compound of the formula:
R₁'' - H
in which R₁'' represents an amino, lower alkylamino or lower alkoxy grouping,
to yield a compound of formula (I) in which R₁ represents an amino, lower alkylamino or lower alkoxy grouping,
- and, if desired, with an alkylating agent such as dimethyl sulphate or an alkyl halide of the formula:
R₂₁ - X
in which R₂₁ represents a lower alkyl grouping and X represents a halogen atom,
to yield a compound of formula (I) in which R₂ represents a lower alkyl grouping,
- or, if desired, with an acid chloride of the formula:
R₂₂Cl
- or an acid anhydride of the formula:
R₂₂OR₂₂,
R₂₂ representing a lower acyl grouping,
to yield a compound of formula (I) in which R₂ represents a lower acyl grouping,
the compounds of formula (I) then, if desired, being either resolved into their optical isomers and then converted into salts by the addition of a pharmaceutically acceptable base or acid, or converted directly into salts in racemic form by the addition of a pharmaceutically acceptable base or acid.

23. Compounds of formula (II): 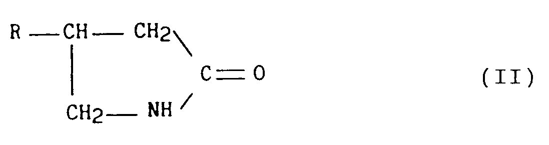 in which R is as defined in claim 1,
it being understood that R cannot represent:
- a 2-benzofuryl grouping that is unsubstituted or substituted in the benzene nucleus by a chlorine atom, a bromine atom or a methoxy group,
- or a 2-benzothienyl grouping,
which can be used as starting materials in the synthesis of the compounds of the invention of formula (I) according to claim 1, their isomers, and also, optionally, their salts of addition with an acid or base.

24. Compounds of formula (III): 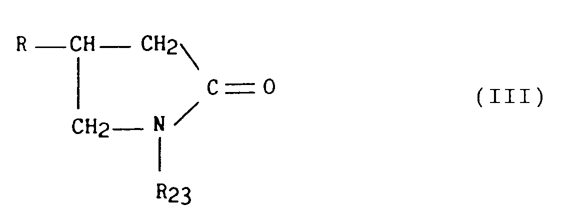 in which R and R₂₃ are as defined in claim 1 and in claim 22, respectively,
which can be used as starting materials in the synthesis of the compounds of the invention of formula (I) according to claim 1,
their isomers, and also, optionally, their salts of addition with an acid or base.

25. Pharmaceutical composition containing as active ingredient at least one compound according to one of claims 1 to 21 in combination with one or more pharmaceutically acceptable non-toxic inert excipients or carriers.

26. Pharmaceutical composition according to claim 25 containing at least one active ingredient according to one of claims 1 to 21 that can be used in the treatment of spastic disorders and disorders connected with senescence.

## Claims (Claims for the following Contracting State(s): ES, GR)

1. Process for the preparation of compounds of the general formula (I): 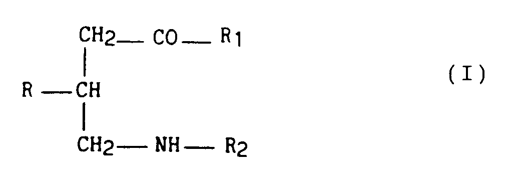 in which:
- R₁ represents a hydroxy, amino, lower alkylamino or lower alkoxy group, or a halogen atom,
- R₂ represents a hydrogen atom, a lower alkyl radical, a lower acyl radical, or a lower alkoxycarbonyl radical,
- R represents:
. a radical of the formula 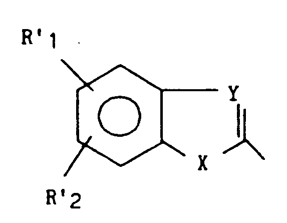 in which:
X represents an oxygen or sulphur atom or an NH grouping,
Y represents a carbon, oxygen or nitrogen atom,
R'₁ and R'₂, which are identical or different, represent a halogen or hydrogen atom or a lower alkyl, lower alkoxy, hydroxy, nitro, amino, lower alkylamino or trifluoromethyl radical,
with the proviso that, when X is an oxygen atom, Y is a carbon atom and each of R'₁ and R₂ is a hydrogen atom, then R'₂ can represent neither a hydrogen atom nor a methoxy group,
. a radical of the formula 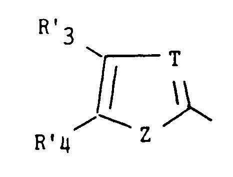 in which:
Z represents an oxygen or sulphur atom or an NH grouping,
T represents a carbon or nitrogen atom,
R'₃ and R'₄, which are identical or different, represent a radical selected from hydrogen, halogen, lower alkyl, lower alkoxy, hydroxy, nitro, amino, lower alkylamino or trifluoromethyl, with the proviso that, when Z is a sulphur atom, T is a carbon atom and R'₃ is a hydrogen atom, R'₄ may not be a methyl grouping or a hydrogen, chlorine or bromine atom, and that, when Z is an oxygen atom, T is a carbon atom and R'₃ is a hydrogen atom, R'₄ may not be a hydrogen atom or a methyl group,
. a cycloalkyl radical having 4 or 5 carbon atoms or a cycloalkylalkyl or dicycloalkylalkyl radical having from 4 to 16 carbon atoms, optionally substituted on the ring by a grouping selected from halogen, hydroxy, lower alkyl, lower alkoxy, nitro, amino, lower alkylamino or trifluoromethyl,
. an optionally substituted aromatic radical having 6 ring members that includes 2 or 3 nitrogen atoms in its carbon skeleton,
. an aromatic radical having 6 ring members that includes from 1 to 3 nitrogen atoms in its carbon skeleton and is fused to a benzene ring, it being optionally possible for each one of these 2 rings to be substituted,
. or a saturated or unsaturated ring having seven ring members that includes one or two nitrogen atoms in its carbon skeleton and is optionally fused to a benzene ring and optionally is substituted on the nitrogen and/or benzene ring,
the term "substituted" indicating that the groupings so qualified can be substituted by one or more halogen atoms or lower alkyl or lower alkoxy, or hydroxy, or trifluoromethyl, or nitro, or amino, or lower alkylamino groupings,
their optical isomers and also, where appropriate, their salts of addition with a pharmaceutically acceptable base or acid,
it being understood that, unless otherwise indicated, the terms "lower alkyl", "lower alkoxycarbonyl", "lower alkoxy", "lower alkylamino" and "lower acyl" indicate groupings containing from 1 to 6 carbon atoms in a straight or branched chain,
characterised in that
there is used as starting material a compound of formula (II): 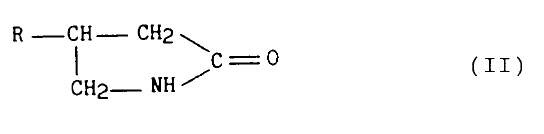 in which R is as defined in formula (I),
which can be:
- either hydrolysed by the action of a metal hydroxide to obtain, after optional purification, a compound of formula (I/a): 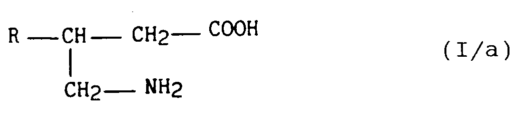
a particular case of the compounds of formula (I) in which R₁ represents a hydroxy grouping, R₂ represents a hydrogen atom and R is as defined in formula (I),
- or converted by the action of a lower alkyl dicarbonate in the presence of a strong base into a compound of formula (III): 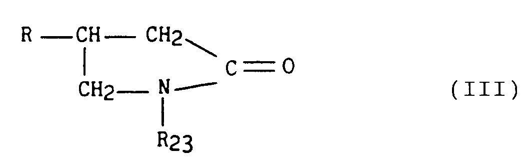
in which R is as defined above and R₂₃ represents a lower alkoxycarbonyl grouping,
which is treated, after optional purification, with an alkali metal hydroxide in an anhydrous medium, and then with an acid to obtain a compound of formula (I/b): 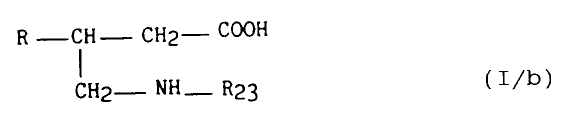 in which R and R₂₃ are as defined above,
a particular case of the compounds of formula (I) in which R₁ represents a hydroxy group, R₂ represents a lower alkoxycarbonyl radical and R is as defined in formula (I),
which, treated in an acidic medium, yields a compound of formula (I/a) as defined above,
which compound of formula (I/a), irrespective of the process by which it has been obtained, may, if desired, be converted by a halogenating agent into its halide of formula (I/c): 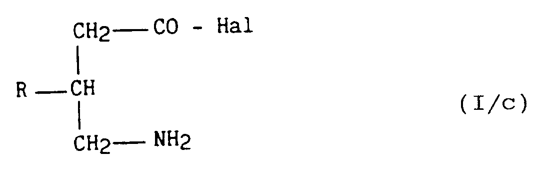 in which Hal represents a halogen atom and R is as defined in formula (I),
a particular case of the compounds of formula (I) in which R₁ represents a halogen atom, R₂ represents a hydrogen atom and R is as defined in formula (I),
which compound of formula (I/a) or (I/c) can be treated, if desired:
- with a compound of the formula:
R₁'' - H
in which R₁'' represents an amino, lower alkylamino or lower alkoxy grouping,
to yield a compound of formula (I) in which R₁ represents an amino, lower alkylamino or lower alkoxy grouping,
- and, if desired, with an alkylating agent such as dimethyl sulphate or an alkyl halide of the formula:
R₂₁ - X
in which R₂₁ represents a lower alkyl grouping and X represents a halogen atom,
to yield a compound of formula (I) in which R₂ represents a lower alkyl grouping,
- or, if desired, with an acid chloride of the formula:
R₂₂Cl
- or an acid anhydride of the formula:
R₂₂OR₂₂_{,}
R₂₂ representing a lower acyl grouping,
to yield a compound of formula (I) in which R₂ represents a lower acyl grouping,
the compounds of formula (I) then, if desired, being either resolved into their optical isomers and then converted into salts by the addition of a pharmaceutically acceptable base or acid, or converted directly into salts in racemic form by the addition of a pharmaceutically acceptable base or acid.

2. Process for the preparation according to claim 1 of compounds of formula (I) in which R represents a radical of the formula: 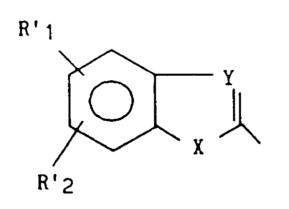 in which R'₁, R'₂, X and Y are as defined in claim 1, their optical isomers and also, where appropriate, their salts of addition with a pharmaceutically acceptable base or acid.

3. Process for the preparation according to claim 1 of compounds of formula (I) in which R represents a radical of the formula: 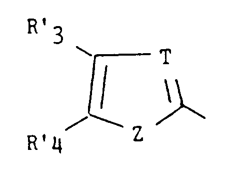 in which Z, T, R'₃ and R'₄ are as defined in claim 1, their optical isomers and also, where appropriate, their salts of addition with a pharmaceutically acceptable acid or base.

4. Process for the preparation according to claim 1 of compounds of formula (I) in which R represents a cycloalkyl radical having 4 or 5 carbon atoms, a cycloalkylalkyl or dicycloalkylalkyl radical having from 4 to 16 carbon atoms, their optical isomers and also, where appropriate, their salts of addition with a pharmaceutically acceptable base or acid.

5. Process for the preparation according to claim 1 of compounds of formula (I) in which R is a radical of the formula: 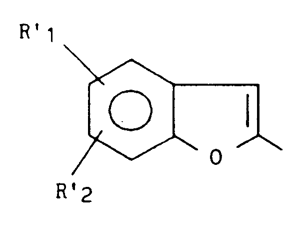 in which R'₁ and R'₂, which are identical or different, represent a halogen or hydrogen atom or a lower alkyl, lower alkoxy or trifluoromethyl radical, with the proviso that, when R'₁ and R₂ are hydrogen atoms, R'₂ can represent neither a hydrogen atom nor a methoxy group, their optical isomers and also, where appropriate, their salts of addition with a pharmaceutically acceptable base or acid.

6. Process for the preparation according to claim 1 of compounds of formula (I) in which R is a radical of the formula: 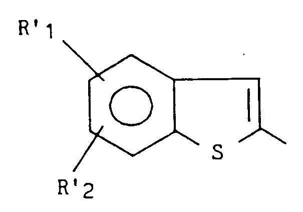 in which R'₁ and R'₂, which are identical or different, represent a hydrogen or halogen atom or a lower alkyl, lower alkoxy or trifluoromethyl radical, their optical isomers and also, where appropriate, their salts of addition with a pharmaceutically acceptable base or acid.

7. Process for the preparation according to claim 1 of compounds of formula (I) in which R represents a grouping: 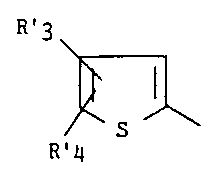 in which R'₃ and R'₄, which are identical or different, represent a radical selected from hydrogen, halogen, lower alkyl, lower alkoxy or trifluoromethyl, with the proviso that, if R'₃ is a hydrogen atom, then R'₄ may not be a hydrogen, chlorine or bromine atom or a methyl grouping,
their optical isomers and also, where appropriate, their salts of addition with a pharmaceutically acceptable base or acid.

8. Process for the preparation according to claim 1 of compounds of formula (I) in which R represents a grouping: 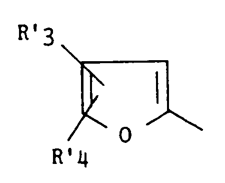 in which R'₃ and R'₄, which are identical or different, represent a radical selected from hydrogen, halogen, lower alkyl, lower alkoxy or trifluoromethyl, with the proviso that, if R'₃ is a hydrogen atom, then R'₄ may not be a hydrogen atom or a methyl grouping,
their optical isomers and also, where appropriate, their salts of addition with a pharmaceutically acceptable base or acid.

9. Process for the preparation according to claim 1 of compounds of formula (I) in which R represents a grouping: 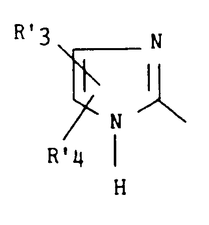 in which R'₃ and R'₄, which are identical or different, represent a radical selected from hydrogen, halogen, lower alkyl, lower alkoxy or trifluoromethyl,
their optical isomers and also, where appropriate, their salts of addition with a pharmaceutically acceptable base or acid.

10. Process for the preparation according to claim 1 of compounds of formula (I) in which R represents:
- an optionally substituted aromatic radical having 6 ring members that includes 2 or 3 nitrogen atoms in its carbon skeleton,
- an aromatic radical having 6 ring members that includes from 1 to 3 nitrogen atoms in its carbon skeleton and is fused to a benzene ring, it being optionally possible for each one of these 2 rings to be substituted,
- or a saturated or unsaturated ring having seven ring members that includes 1 or 2 nitrogen atoms in its carbon skeleton and is optionally fused to a benzene ring and optionally is substituted on the nitrogen and/or benzene ring,
the term "substituted" indicating that the groupings so qualified can be substituted by one or more halogen atoms or lower alkyl or lower alkoxy or hydroxy or trifluoromethyl or amino or lower alkylamino or nitro groupings,
their isomers and also, where appropriate, their salts of addition with a pharmaceutically acceptable acid or base.

11. Process for the preparation according to claim 1 of compounds of formula (I) in which R represents an indolyl, pyrimidinyl, pyridazinyl, 1,3,5-triazinyl or azepinyl radical optionally substituted by a lower alkyl, lower alkoxy or trifluoromethyl grouping or by one or more halogen atoms, their isomers and also, where appropriate, their salts of addition with a pharmaceutically acceptable acid or base.

12. Process for the preparation according to claim 1 of the compound which is 3-(2-imidazolyl)-4-aminobutanoic acid, its isomers and also its salts of addition with a pharmaceutically acceptable acid or base.

13. Process for the preparation according to claim 1 of the compound which is 3-(2-(5-isopropylbenzofuryl))-4-aminobutanoic acid, its isomers and also its salts of addition with a pharmaceutically acceptable acid or base.

14. Process for the preparation according to claim 1 of the compound which is 3-(2-(5-ethylbenzofuryl))-4-aminobutanoic acid, its isomers and also its salts of addition with a pharmaceutically acceptable acid or base.

15. Process for the preparation according to claim 1 of the compound which is 3-(2-(5-(1-methylpropyl)-benzofuryl))-4-aminobutanoic acid, its isomers and also its salts of addition with a pharmaceutically acceptable acid or base.

16. Process for the preparation according to claim 1 of the compound which is 3-(2-(5-fluorobenzofuryl))-4-aminobutanoic acid, its isomers and also its salts of addition with a pharmaceutically acceptable acid or base.

17. Process for the preparation according to claim 1 of the compound which is 3-(2-(4,5-dichlorothienyl))-4-aminobutanoic acid, its isomers and also its salts of addition with a pharmaceutically acceptable acid or base.

18. Process for the preparation according to claim 1 of the compound which is 3-(2-(5-chlorobenzofuryl))-4-aminobutanoic acid, its isomers and also its salts of addition with a pharmaceutically acceptable acid or base.

19. Process for the preparation according to claim 1 of the compound which is 3-(2-benzothienyl)-4-aminobutanoic acid, its isomers and also its salts of addition with a pharmaceutically acceptable acid or base.

20. Process for the preparation according to claim 1 of the compound which is 3-(-2-(5-methylbenzofuryl))-4-aminobutanoic acid, its isomers and also its salts of addition with a pharmaceutically acceptable acid or base.

21. Process for the preparation according to claim 1 of the compound which is 3-(2-(5-ethoxybenzofuryl))-4-aminobutanoic acid, its isomers and also its salts of addition with a pharmaceutically acceptable acid or base.

22. Process for the preparation of the compounds of formula (II): 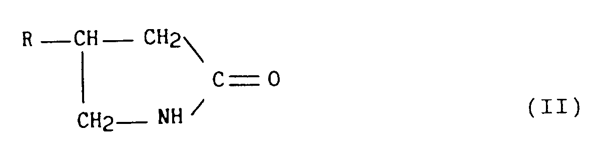 in which R is as defined in claim 1,
it being understood that R cannot represent:
- a 2-benzofuryl grouping that is unsubstituted or substituted in the benzene nucleus by a chlorine atom, a bromine atom or a methoxy group,
- or a 2-benzothienyl grouping,
their isomers, and also, optionally, their salts of addition with an acid or base,
characterised in that
- either an aldehyde of formula (V):
R - CHO (V)
in which R is as defined above,
is condensed in an aprotic non-polar solvent with a carboxymethylidenetriphenylphosphorane ester of formula (VI):
(C₆H₅)₃ - P = CH - COOR' (VI)
in which R' represents a lower alkyl radical,
to obtain an ester of formula (VII):
R - CH = CH - COOR' (VII)
in which R and R' are as defined above,
which is condensed in a protic polar medium with nitromethane in the presence of a strong base, to obtain a compound of formula (VIII): 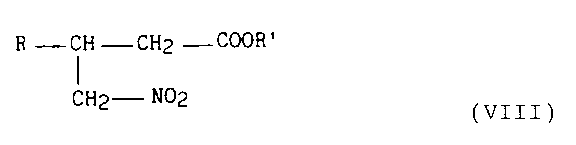 in which R and R' are as defined above,
which is reduced in an alcoholic medium by the action of hydrogen in the presence of a metal catalyst to form a compound of formula (IX): 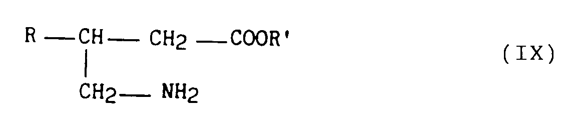 in which R and R' are as defined above,
which is cyclised by heating to form a compound of formula (II): 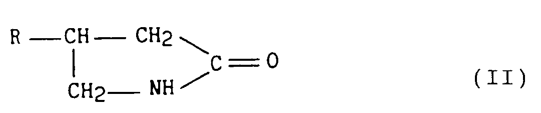 in which R is as defined above,
- or a compound of formula (X):
RCOCH₃ (X)
in which R is as defined above,
is treated at elevated temperature and in the presence of zinc with a compound of formula (XI):
Br - CH₂ - COOA (XI)
in which A represents a lower alkyl grouping,
to yield, after optional acidic hydrolysis, extraction and purification, a compound of formula (XII): 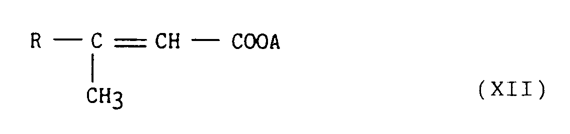 in which R and A are as defined above,
which is treated with N-bromosuccinimide to yield a compound of formula (XIII): 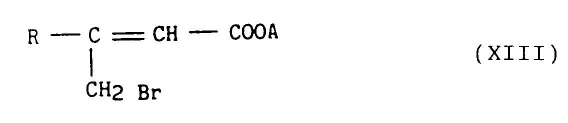 in which R and A are as defined above,
which is treated with ammonia, preferably an excess thereof, to yield a compound of formula (XIV): 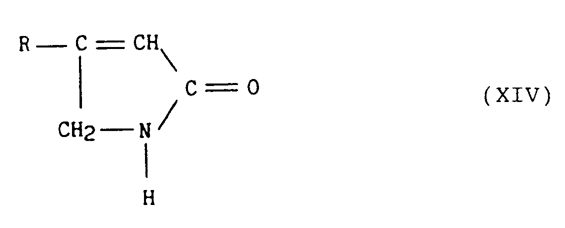 in which R is as defined above,
which is subjected to catalytic hydrogenation to obtain a compound of formula (II) as defined above.
23. Process for the preparation of pharmaceutical compositions containing as active ingredient at least one compound prepared in accordance with one of claims 1 to 21 in combination with one or more pharmaceutically acceptable non-toxic inert excipients or carriers.
24. Process for the preparation of pharmaceutical compositions according to claim 23 containing at least one active ingredient prepared according to one of claims 1 to 21 that can be used in the treatment of spastic disorders and disorders connected with senescence.

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel (I): 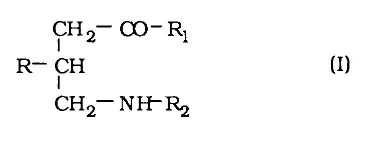 in der:
- R₁ eine Hydroxylgruppe. Aminogruppe, niedrigmolekulare Aminoalkylgruppe oder niedrigmolekulare Alkoxygruppe oder ein Halogenatom,
- R₂ ein Wasserstoffatom, eine niedrigmolekulare Alkylgruppe, eine niedrigmolekulare Acylgruppe oder eine niedrigmolekulare Alkoxycarbonylgruppe,
- R:
. eine Gruppe der Formel: 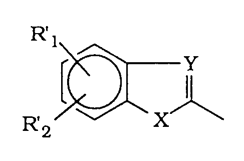 in der:
X ein Sauerstoffatom, ein Schwefelatom oder eine NH-Gruppe,
Y ein Kohlenstoffatom, ein Sauerstoffatom oder ein Stickstoffatom,
R'₁ und R'₂, die gleichartig oder verschieden sein können, ein Halogenatom oder ein Wasserstoffatom oder eine niedrigmolekulare Alkylgruppe oder eine niedrigmolekulare Alkoxygruppe, eine Hydroxylgruppe, eine Nitrogruppe, eine Aminogruppe, eine niedrigmolekulare Alkylaminogruppe oder eine Trifluormethylgruppe,
mit der Maßgabe darstellen, daß wenn X ein Sauerstoffatom, Y ein Kohlenstoffatom und R'₁ und R₂ jeweils ein Wasserstoffatom bedeuten, R'₂ weder ein Wasserstoffatom noch eine Methoxygruppe darstellt,
. eine Gruppe der Formel 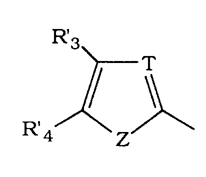 in der:
Z ein Sauerstoffatom, ein Schwefelatom oder eine NH-Gruppe,
T ein Kohlenstoffatom oder ein Stickstoffatom und
R'₃ und R'₄, die gleichartig oder verschieden sein können, eine Gruppe ausgewählt aus Wasserstoff, Halogen, niedrigmolekularen Alkyl- oder Alkoxygruppen, Hydroxylgruppen, Nitrogruppen, Aminogruppen, niedrigmolekularen Alkylaminogruppen oder Trifluormethylgruppen mit der Maßgabe darstellen, daß wenn Z ein Schwefelatom, T ein Kohlenstoffatom und R'₃ ein Wasserstoffatom bedeuten, R'₄ keine Methylgruppe oder ein Wasserstoffatom, ein Chloratom oder ein Bromatom darstellt, und wenn Z ein Sauerstoffatom, T ein Kohlenstoffatom und R'₃ ein Wasserstoffatom bedeuten, R'₄ kein Wasserstoffatom oder eine Methylgruppe darstellt,
. eine Cycloalkylgruppe mit 4 oder 5 Kohlenstoffatomen, eine Cycloalkylalkyl- oder Dicycloalkylalkylgruppe mit 4 bis 16 Kohlenstoffatomen, die gegebenenfalls am Ring durch eine Gruppe ausgewählt aus Halogenatomen, Hydroxylgruppen, niedrigmolekularen Alkyl- oder Alkoxygruppen, Nitrogruppen, Aminogruppen, niedrigmolekularen Alkylaminogruppen oder Trifluormethylgruppen substituiert sind,
. eine aromatische Gruppe mit 6 Gliedern, die gegebenenfalls substituiert ist und in ihrem Kohlenstoffskelett 2 oder 3 Stickstoffatome aufweist,
. eine aromatische Gruppe mit 6 Gliedern, die in ihrem Kohlenstoffskelett 1 bis 3 Stickstoffatome aufweist und mit einem Benzolring verbunden ist, wobei diese beiden Ringe gegebenenfalls substituiert sein können,
. oder einen Ring mit sieben Gliedern, der gesättigt ist oder nicht und in seinem Kohlenstoffskelett ein oder zwei Stickstoffatome aufweist und gegebenenfalls mit einem Benzolring verbunden ist und gegebenenfalls am stickstoffhaltigen Ring und/oder am Benzolring substituiert ist,
bedeuten, wobei der Begriff ''substituiert" bedeutet, daß die damit gekennzeichneten Gruppen durch ein oder mehrere Halogenatome oder niedrigmolekulare Alkylgruppen oder niedrigmolekulare Alkoxygruppen oder Hydroxylgruppen oder Trifluormethylgruppen oder Nitrogruppen oder Aminogruppen oder niedrigmolekulare Alkylaminogruppen substituiert sein können,
deren optische Isomere sowie gegebenenfalls deren Additionssalze mit einer pharmazeutisch annehmbaren Base oder Säure,
wobei es sich, wenn nichts anderes angegeben ist, versteht, daß die Begriffe "niedrigmolekulare Alkylgruppe", "niedrigmolekulare Alkoxycarbonylgruppe", "niedrigmolekulare Alkoxygruppe", "niedrigmolekulare Alkylaminogruppe"- und "niedrigmolekulare Acylgruppe" dafür stehen, daß die Gruppen 1 bis 6 Kohlenstoffatome in gerader oder verzweigter Kette enthalten.

2. Verbindungen der Formel (I) nach Anspruch 1, worin R eine Gruppe der Formel: 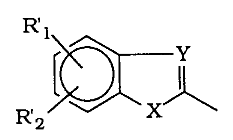 darstellt, in der R'₁, R'₂, X und Y die in Anspruch 1 angegebenen Bedeutungen besitzen, deren optische Isomere sowie gegebenenfalls deren Additionssalze mit einer pharmazeutisch annehmbaren Base oder Säure.

3. Verbindungen der Formel (I) nach Anspruch 1, worin R eine Gruppe der Formel: 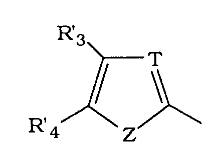 darstellt, worin Z, T, R'₃ und R'₄ die in Anspruch 1 angegebenen Bedeutungen besitzen, deren optische Isomere sowie gegebenenfalls deren Additionssalze mit einer pharmazeutisch annehmbarne Säure oder Base.

4. Verbindungen der Formel (I) nach Anspruch 1, worin R eine Cycloalkylgruppe mit 4 oder 5 Kohlenstoffatomen, eine Cycloalkylalkyl- oder Dicycloalkylalkylgruppe mit 4 bis 16 Kohlenstoffatomen bedeutet, deren optische Isomere sowie gegebenenfalls deren Additionssalze mit einer pharmazeutisch annehmbaren Base oder Säure.

5. Verbindungen der Formel (I) nach Anspruch 1, worin R eine Gruppe der Formel: 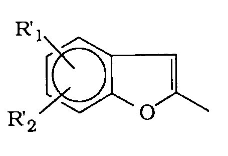 darstellt, worin R'₁ und R'₂, die gleichartig oder verschieden sein können, ein Halogenatom oder ein Wasserstoffatom oder eine niedrigmolekulare Alkylgruppe, niedrigmolekulare Alkoxygruppe oder eine Trifluormethylgruppe mit der Maßgabe bedeuten, daß, wenn R'₁ und R₂ Wasserstoffatome darstellen, R'₂ weder ein Wasserstoffatom noch eine Methoxygruppe bedeutet, deren optische Isomere sowie gegebenenfalls deren Additionssalze mit einer pharmazeutisch annehmbaren Base oder Säure.

6. Verbindungen der Formel (I) nach Anspruch 1, worin R eine Gruppe der Formel: 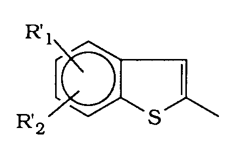 darstellt, in der R'₁ und R'₂, die gleichartig oder verschieden sein können, ein Wasserstoff- oder Halogenatom oder eine niedrigmolekulare Alkylgruppe, eine niedrigmolekulare Alkoxygruppe oder eine Trifluormethylgruppe bedeuten, deren optische Isomere sowie gegebenenfalls deren Additionssalze mit einer pharmazeutisch annehmbaren Base oder Säure.

7. Verbindungen nach Anspruch 1, worin R eine Gruppe: 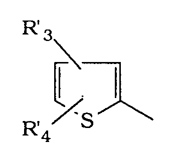 darstellt, worin R'₃ und R'₄, die gleichartig oder verschieden sein können, eine Gruppe ausgewählt aus Wasserstoff, Halogen, niedrigmolekularen Alkylgruppen, niedrigmolekularen Alkoxygruppen oder Trifluormethylgruppen mit der Maßgabe bedeuten, daß, wenn R'₃ ein Wasserstoffatom darstellt, R'₄ kein Wasserstoffatom, Chloratom oder Bromatom oder eine Methylgruppe bedeuten kann, deren optische Isomere sowie gegebenenfalls deren Additionssalze mit einer pharmazeutisch annehmbaren Base oder Säure.

8. Verbindungen nach Anspruch 1, worin R eine Gruppe: 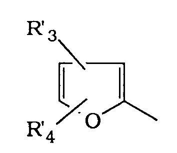 darstellt, worin R'₃ und R'₄, die gleichartig oder verschieden sein können, eine Gruppe ausgewählt aus Wasserstoff, Halogen, niedrigmolekularen Alkylgruppen, niedrigmolekularen Alkoxygruppen oder Trifluormethylgruppen mit der Maßgabe bedeuten, daß, wenn R'₃ ein Wasserstoffatom darstellt, R'₄ kein Wasserstoffatom oder eine Methylgruppe bedeuten kann, deren optische Isomere sowie gegebenenfalls deren Additionssalze mit einer pharmazeutisch annehmbaren Base oder Säure.

9. Verbindungen nach Anspruch 1, worin R eine Gruppe: 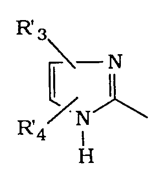 darstellt, worin R'₃ und R'₄, die gleichartig oder verschieden sein können, eine Gruppe ausgewählt aus Wasserstoff, Halogen, niedrigmolekularen Alkylgruppen, niedrigmolekularen Alkoxygruppen und Trifluormethylgruppen bedeuten, deren optische Isomere sowie gegebenenfalls deren Additionssalze mit einer pharmazeutisch annehmbaren Base oder Säure.

10. Verbindungen nach Anspruch 1, worin R:
- eine aromatische Gruppe mit 6 Gliedern, die gegebenenfalls substituiert ist und in ihrem Kohlenstoffskelett 2 oder 3 Stickstoffatome aufweist,
- eine aromatische Gruppe mit 6 Gliedern, die In ihrem Kohlenstoffskelett 1 bis 3 Stickstoffatome aufweist und mit einem Benzolring verbunden ist, wobei diese beiden Ringe gegebenenfalls substituiert sein können,
- oder einen Ring mit sieben Gliedern, der gesättigt ist oder nicht und in seinem Kohlenstoffskelett 1 oder 2 Stickstoffatome aufweist und gegebenenfalls mit einem Benzolring verbunden ist und gegebenenfalls am stickstoffhaltigen Ring und/oder am Benzolring substituiert ist,
bedeutet,
wobei der Begriff "substituiert" für die damit gekennzeichneten Gruppen bedeutet, daß diese durch eines oder mehrere Halogenatome oder niedrigmolekulare Alkylgruppen oder niedrigmolekulare Alkoxygruppen oder Hydroxylgruppen oder Trifluormethylgruppen oder Aminogruppen oder niedrigmolekulare Alkylaminogruppen oder Nitrogruppen substituiert sein können,
deren Isomere sowie deren Additionssalze mit einer pharmazeutisch annehmbaren Säure oder Base.

11. Verbindungen nach Anspruch 1, worin R eine Indolyl-, Pyrimidinyl-, Pyridazinyl-, 1, 3, 5-Triazinyl- oder Azepinyl-gruppe darstellt, die gegebenenfalls durch eine niedrigmolekulare Alkylgruppe, niedrigmolekulare Alkoxygruppe, eine Trifluormethylgruppe oder durch eines oder mehrere Halogenatome substituiert ist, deren Isomere sowie deren Additionssalze mit einer pharmazeutisch annehmbaren Säure oder Base.

12. Verbindung nach Anspruch 1, nämlich 3-(2-Imidazolyl) -4-amino-butansäure, deren Isomere sowie deren Additionssalze mit einer pharmazeutisch annehmbaren Säure oder Base.

13. Verbindung nach Anspruch 1, nämlich 3-(2-(5-Isopropyl-benzofuryl))-4-amino-butansäure, deren Isomere sowie deren Additionssalze mit einer pharmazeutisch annehmbaren Säure oder Base.

14. Verbindung nach Anspruch 1, nämlich 3-(2-(5-Ethyl-benzofuryl))-4-amino-butansäure, deren Isomere sowie deren Additionssalze mit einer pharmazeutisch annehmbaren Säure oder Base.

15. Verbindung nach Anspruch 1, nämlich 3-(2-(5-( 1 Methylpropyl)-benzofuryl))-4-amino-butansäure, deren Isomere sowie deren Additionssalze mit einer pharmazeutisch annehmbaren Säure oder Base.

16. Verbindung nach Anspruch 1, nämlich 3-(2-(5-Fluor-benzofuryl))-4-amino-butansäure, deren Isomere sowie deren Additionssalze mit einer pharmazeutisch annehmbaren Säure oder Base.

17. Verbindung nach Anspruch 1, nämlich 3-(2-(4,5-Dichlor-thienyl))-4-amino-butansäure, deren Isomere sowie deren Additionssalze mit einer pharmazeutisch annehmbaren Säure oder Base.

18. Verbindung nach Anspruch 1, nämlich 3-(2-(5-Chlor-benzofuryl))-4-amino-butansäure, deren Isomere sowie deren Additionssalze mit einer pharmazeutisch annehmbaren Säure oder Base.

19. Verbindung nach Anspruch 1, nämlich 3-(2-Benzothienyl)-4-amino-butansäure, deren Isomere sowie deren Additionssalze mit einer pharmazeutisch annehmbaren Säure oder Base.

20. Verbindung nach Anspruch 1, nämlich 3-(2-(5-Methyl-benzofuryl))-4-amino-butansäure, deren Isomere sowie deren Additionssalze mit einer pharmazeutisch annehmbaren Säure oder Base.

21. Verbindung nach Anspruch 1, nämlich 3-(2-(5-Ethoxy-benzofuryl))-4-amino-butansäure - deren Isomere sowie deren Additionssalze mit einer pharmazeutisch annehmbaren Säure oder Base.

22. Verfahren zur Herstellung der Verbindungen der Formel (I), **dadurch gekennzeichnet**, daß man als Ausgangsmaterial eine Verbindung der Formel (II): 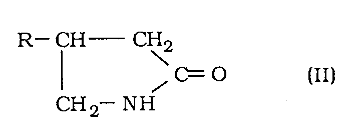 in der R die bezüglich der Formel (I) angegebenen Bedeutungen besitzt, verwendet, welche man:
- entweder durch Einwirkung eines Metallhydroxids hydrolysieren kann, so daß man nach der eventuellen Reinigung eine Verbindung der Formel (I/a): 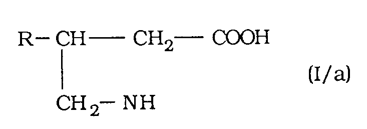 erhält, einem Sonderfall der Verbindungen der Formel I, worin R₁ eine Hydroxylgruppe, R₂ ein Wasserstoffatom bedeuten und R die bezüglich der Formel (I) angegebenen Bedeutungen besitzt,
- oder durch Einwirkung eines niedrigmolekularen Alkyldicarbonats in Gegenwart einer starken Base in eine Verbindung der Formel (III): 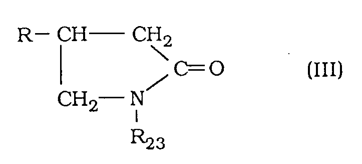 umwandeln kann, in der R die oben angegebenen Bedeutungen besitzt und R₂₃ eine niedrigmolekulare Alkoxycarbonylgruppe darstellt,
welche man nach der eventuellen Reinigung mit einem Alkalimetallhydroxid in wasserfreiem Medium und dann mit einer Säure behandelt zur Bildung einer Verbindung der Formel (I/b): 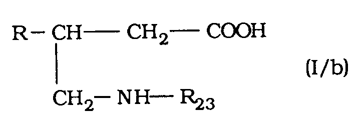 worin R und R₂₃ die oben angegebenen Bedeutungen besitzen,
einem Sonderfall der Verbindungen der Formel (I), worin R₁ eine Hydroxylgruppe und R₂ eine niedrigmolekulare Alkoxycarbonylgruppe bedeuten und R die bezüglich der Formel (I) angegebenen Bedeutungen besitzt,
welche bei der Behandlung in saurem Medium zu einer Verbindung der Formel (I/a) führt, wie sie oben definiert worden ist,
welche Verbindung der Formel (I/a), nach welchem Verfahren man sie auch hergestellt hat, man gewünschtenfalls mit einem Halogenierungsmittel in ihr Halogenid der Formel (I/c): 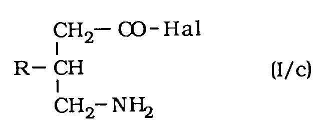 umwandeln kann, worin Hal ein Halogenatom darstellt und R die bezüglich der Formel (I) angegebenen Bedeutungen besitzt,
einem Sonderfall der Verbindungen der Formel (I), worin R₁ ein Halogenatom und R₂ ein Wasserstoffatom darstellen und R die bezüglich der Formel (I) angegebenen Bedeutungen besitzt,
welche Verbindung der Formel (I/a) oder (I/c) man gewünschtenfalls
- mit einer Verbindung der Formel:
R₁'' - H
in der R₁'' eine Aminogruppe, niedrigmolekulare Alkylaminogruppe oder niedrigmolekulare Alkoxygruppe darstellt, behandeln kann, zur Bildung eine Verbindung der Formel (I), worin R₁ eine Aminogruppe, eine niedrigmolekulare Alkylaminogruppe oder eine niedrigmolekulare Alkoxygruppe darstellt,
- und gewünschtenfalls mit einem Alkylierungsmittel, wie Dimethylsulfat oder einem Alkylhalogenid der Formel:
R₂₁ - X
worin R₂₁ eine niedrigmolekulare Alkylgruppe darstellt und X ein Halogenatom bedeutet,
behandeln kann, zur Bildung einer Verbindung der Formel (I), worin R₂ eine niedrigmolekulare Alkylgruppe darstellt,
- oder die man schließlich gewünschtenfalls mit einem Säurechlorid der Formel:
R₂₂Cl
oder einem Säureanhydrld der Formel:
R₂₂OR₂₂
worin R₂₂ eine niedrigmolekulare Acylgruppe darstellt,
behandeln kann, zur Bildung einer Verbindung der Formel (I), in der R₂ eine niedrigmolekulare Acylgruppe darstellt,
welche Verbindungen der Formel (I) man anschließend gewünschtenfalls in ihre optischen Isomeren aufspaltet und durch Zugabe einer pharmazeutisch annehmbaren Base oder Säure in ihre Salze überführt oder direkt in racemischer Form durch Zugabe einer pharmazeutisch annehmbaren Base oder Säure in die Salze überführt.

23. Verbindungen der Formel (II): 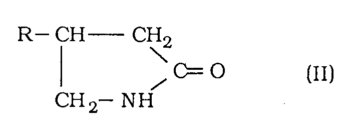 worin R die in Anspruch 1 angegebenen Bedeutungen besitzt,
wobei es sich versteht, daß R:
- keine 2-Benzofurylgruppe, die unsubstituiert ist oder am Benzolkern durch ein Chloratom, ein Bromatom oder eine Methoxygruppe substituiert ist,
- oder keine 2-Benzothienylgruppe bedeutet,
welche Verbindungen geeignet sind als Ausgangsmaterialien für die Synthese der Verbindungen der Formel (I) nach Anspruch 1, deren Isomere sowie gegebenenfalls deren Additionssalze mit einer Säure oder Base.

24. Verbindungen der Formel (III): 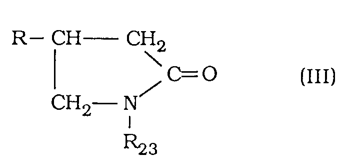 in der R und R₂₃ die in Anspruch 1 bzw. Anspruch 22 angegebenen Bedeutungen besitzen,
welche Verbindungen als Ausgangsmaterialien für die Synthese der erfindungsgemäßen Verbindungen der Formel (I) nach Anspruch 1 geeignet sind,
deren Isomere sowie gegebenenfalls deren Additionssalze mit einer Säure oder einer Base.

25. Pharmazeutische Zubereitung enthaltend als Wirkstoff mindestens eine Verbindung nach einem der Ansprüche 1 bis 21 in Kombination mit einem oder mehreren inerten, nichttoxischen, pharmazeutisch annehmbaren Trägermaterialien oder Bindemitteln.

26. Pharmazeutische Zubereitung nach Anspruch 25, enthaltend mindestens einen Wirkstoff nach einem der Ansprüche 1 bis 21 zur Behandlung von spastischen Störungen und Störungen des Alterns.

## Patentansprüche (Patentansprüche für folgende(n) Vertragsstaat(en): ES, GR)

1. Verfahren zur Herstellung der Verbindungen der allgemeinen Formel (I): 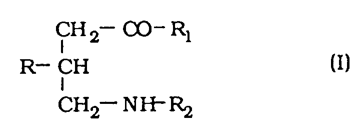 in der:
- R₁ eine Hydroxylgruppe, Aminogruppe, niedrigmolekulare Aminoalkylgruppe oder niedrigmolekulare Alkoxygruppe oder ein Halogenatom,
- R₂ ein Wasserstoffatom, eine niedrigmolekulare Alkylgruppe, eine niedrigmolekulare Acylgruppe oder eine niedrigmolekulare Alkoxycarbonylgruppe,
- R:
. eine Gruppe der Formel: 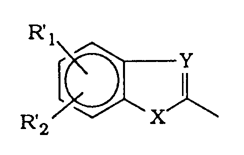 in der:
X ein Sauerstoffatom, ein Schwefelatom oder eine NH-Gruppe,
Y ein Kohlenstoffatom, ein Sauerstoffatom oder ein Stickstoffatom,
R'₁ und R'₂, die gleichartig oder verschieden sein können, ein Halogenatom oder ein Wasserstoffatom oder eine niedrigmolekulare Alkylgruppe oder eine niedrigmolekulare Alkoxygruppe, eine Hydroxylgruppe, eine Nitrogruppe, eine Aminogruppe, eine niedrigmolekulare Alkylaminogruppe oder eine Trifluormethylgruppe,
mit der Maßgabe darstellen, daß wenn X ein Sauerstoffatom, Y ein Kohlenstoffatom und R'₁ und R₂ jeweils ein Wasserstoffatom bedeuten, R'₂ weder ein Wasserstoffatom noch eine Methoxygruppe darstellt,
. eine Gruppe der Formel 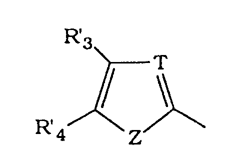 in der:
Z ein Sauerstoffatom, ein Schwefelatom oder eine NH-Gruppe,
T ein Kohlenstoffatom oder ein Stickstoffatom und
R'₃ und R'₄, die gleichartig oder verschieden sein können, eine Gruppe ausgewählt aus Wasserstoff, Halogen' niedrigmolekularen Alkyl- oder Alkoxygruppen, Hydroxylgruppen, Nitrogruppen, Aminogruppen, niedrigmolekularen Alkylaminogruppen oder Trifluormethylgruppen mit der Maßgabe darstellen, daß wenn Z ein Schwefelatom, T ein Kohlenstoffatom und R'₃ ein Wasserstoffatom bedeuten, R'₄ keine Methylgruppe oder ein Wasserstoffatom, ein Chloratom oder ein Bromatom darstellt, und wenn Z ein Sauerstoffatom, T ein Kohlenstoffatom und R'₃ ein Wasserstoffatom bedeuten, R'₄ kein Wasserstoffatom oder eine Methylgruppe darstellt,
. eine Cycloalkylgruppe mit 4 oder 5 Kohlenstoffatomen, eine Cycloalkylalkyl- oder Dicycloalkylalkylgruppe mit 4 bis 16 Kohlenstoffatomen, die gegebenenfalls am Ring durch eine Gruppe ausgewählt aus Halogenatomen, Hydroxylgruppen, niedrigmolekularen Alkyl- oder Alkoxygruppen, Nitrogruppen, Aminogruppen, niedrigmolekularen Alkylaminogruppen oder Trifluormethylgruppen substituiert sind,
. eine aromatische Gruppe mit 6 Gliedern, die gegebenenfalls substituiert ist und in ihrem Kohlenstoffskelett 2 oder 3 Stickstoffatome aufweist,
. eine aromatische Gruppe mit 6 Gliedern, die in ihrem Kohlenstoffskelett 1 bis 3 Stickstoffatome aufweist und mit einem Benzolring verbunden ist, wobei diese beiden Ringe gegebenenfalls substituiert sein können,
. oder einen Ring mit sieben Gliedern, der gesättigt ist oder nicht und in seinem Kohlenstoffskelett ein oder zwei Stickstoffatome aufweist und gegebenenfalls mit einem Benzolring verbunden ist und gegebenenfalls am stickstoffhaltigen Ring und/oder am Benzolring substituiert ist,
bedeuten, wobei der Begriff "substituiert" bedeutet, daß die damit gekennzeichneten Gruppen durch ein oder mehrere Halogenatome oder niedrigmolekulare Alkylgruppen oder niedrigmolekulare Alkoxygruppen oder Hydroxylgruppen oder Trifluormethylgruppen oder Nitrogruppen oder Aminogruppen oder niedrigmolekulare Alkylaminogruppen substituiert sein können,
von deren optischen Isomeren sowie gegebenenfalls von deren Additionssalzen mit einer pharmazeutisch annehmbaren Base oder Säure,
wobei es sich, wenn nichts anderes angegeben ist, versteht, daß die Begriffe "niedrigmolekulare Alkylgruppe", "niedrigmolekulare Alkoxycarbonylgruppe", "niedrigmolekulare Alkoxygruppe", "niedrigmolekulare Alkylaminogruppe" und "niedrigmolekulare Acylgruppe" dafür stehen, daß die Gruppen 1 bis 6 Kohlenstoffatome in gerader oder verzweigter Kette enthalten,
**dadurch gekennzeichnet**, daß man als Ausgangsmaterial eine Verbindung der Formel (II): 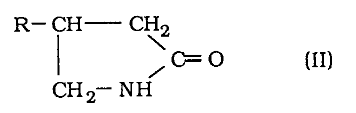 in der R die bezüglich der Formel (I) angegebenen Bedeutungen besitzt, verwendet, welche man:
- entweder durch Einwirkung eines Metallhydroxids hydrolysieren kann, so daß man nach der eventuellen Reinigung eine Verbindung der Formel (I/a): 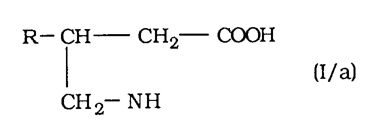 erhält, einem Sonderfall der Verbindungen der Formel I, worin R₁ eine Hydroxylgruppe, R₂ ein Wasserstoffatom bedeuten und R die bezüglich der Formel (I) angegebenen Bedeutungen besitzt,
- oder durch Einwirkung eines niedrigmolekularen Alkyldicarbonats in Gegenwart einer starken Base in eine Verbindung der Formel (III): 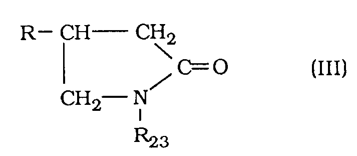 umwandeln kann, In der R die oben angegebenen Bedeutungen besitzt und R₂₃ eine niedrigmolekulare Alkoxycarbonylgruppe darstellt,
welche man nach der eventuellen Reinigung mit einem Alkalimetallhydroxid in wasserfreiem Medium und dann mit einer Säure behandelt zur Bildung einer Verbindung der Formel (I/b): 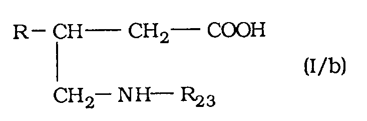 worin R und R₂₃ die oben angegebenen Bedeutungen besitzen,
einem Sonderfall der Verbindungen der Formel (I), worin R₁ eine Hydroxylgruppe und R₂ eine niedrigmolekulare Alkoxycarbonylgruppe bedeuten und R die bezüglich der Formel (I) angegebenen Bedeutungen besitzt.
welche bei der Behandlung in saurem Medium zu einer Verbindung der Formel (I/a) führt, wie sie oben definiert worden ist,
welche Verbindung der Formel (I/a), nach welchem Verfahren man sie auch hergestellt hat, man gewünschtenfalls mit einem Halogenierungsmittel in ihr Halogenid der Formel (I/c): 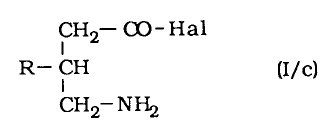 umwandeln kann, worin Hal ein Halogenatom darstellt und R die bezüglich der Formel (I) angegebenen Bedeutungen besitzt,
einem Sonderfall der Verbindungen der Formel (I), worin R₁ ein Halogenatom und R₂ ein Wasserstoffatom darstellen und R die bezüglich der Formel (I) angegebenen Bedeutungen besitzt,
welche Verbindung der Formel (I/a) oder (I/c) man gewünschtenfalls
- mit einer Verbindung der Formel:
R₁'' - H
in der R₁'' eine Aminogruppe, niedrigmolekulare Alkylaminogruppe oder niedrigmolekulare Alkoxygruppe darstellt, behandeln kann, zur Bildung eine Verbindung der Formel (I), worin R₁ eine Aminogruppe, eine niedrigmolekulare Alkylaminogruppe oder eine niedrigmolekulare Alkoxygruppe darstellt,
- und gewünschtenfalls mit einem Alkylierungsmittel, wie Dimethylsulfat oder einem Alkylhalogenid der Formel:
R₂₁ - X
worin R₂₁ eine niedrigmolekulare Alkylgruppe darstellt und X ein Halogenatom bedeutet,
behandeln kann, zur Bildung einer Verbindung der Formel (I), worin R₂ eine niedrigmolekulare Alkylgruppe darstellt,
- oder die man schließlich gewünschtenfalls mit einem Säurechlorid der Formel:
R₂₂Cl
oder einem Säureanhydrid der Formel:
R₂₂OR₂₂
worin R₂₂ eine niedrigmolekulare Acylgruppe darstellt,
behandeln kann, zur Bildung einer Verbindung der Formel (I), in der R₂ eine niedrigmolekulare Acylgruppe darstellt,
welche Verbindungen der Formel (I) man anschließend gewünschtenfalls in ihre optischen Isomeren aufspaltet und durch Zugabe einer pharmazeutisch annehmbaren Base oder Säure in ihre Salze überführt oder direkt in racemischer Form durch Zugabe einer pharmazeutisch annehmbaren Base oder Säure in die Salze überführt.

2. Verfahren nach Anspruch 1 zur Herstellung der Verbindungen der Formel (I), worin R eine Gruppe der Formel: 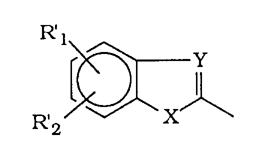 darstellt, in der R'₁, R'₂, X und Y die in Anspruch 1 angegebenen Bedeutungen besitzen, sowie von deren optischen Isomeren sowie gegebenenfalls von deren Additionssalzen mit einer pharmazeutisch annehmbaren Base oder Säure.

3. Verfahren nach Anspruch 1 zur Herstellung der Verbindungen der Formel (I), in der Reine Gruppe der Formel: 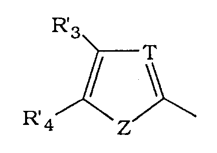 darstellt, worin Z, T, R'₃ und R'₄ die in Anspruch 1 angegebenen Bedeutungenbesitzen, von deren optischen Isomeren sowie gegebenenfalls von deren Additionssalzen mit einer pharmazeutisch annehmbarne Säure oder Base.

4. Verfahren nach Anspruch 1 zur Herstellung der Verbindungen der Formel (I), worin R eine Cycloalkylgruppe mit 4 oder 5 Kohlenstoffatomen, eine Cycloalkylalkyl- oder Dicycloalkyl-alkylgruppe mit 4 bis 16 Kohlenstoffatomen bedeutet, von deren deren optischen Isomeren sowie gegebenenfalls von deren Additionssalzen mit einer pharmazeutisch annehmbaren Base oder Säure.

5. Verfahren nach Anspruch 1 zur Herstellung von Verbindungen der Formel (I), worin R eine Gruppe der Formel: 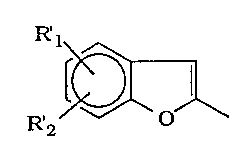 darstellt, worin R'₁ und R'₂ die gleichartig oder verschieden sein können, ein Halogenatom oder ein Wasserstoffatom oder eine niedrigmolekulare Alkylgruppe, niedrigmolekulare Alkoxygruppe oder eine Trifluormethylgruppe mit der Maßga be bedeuten, daß, wenn R'₁ und R₂ Wasserstoffatome darstellen, R'₂ weder ein Wasserstoffatom noch eine Methoxygruppe bedeutet,
von deren optischen Isomeren sowie gegebenenfalls von deren Additionssalzen mit einer pharmazeutisch annehmbaren Base oder Säure.

6. Verfahren nach Anspruch 1 zur Herstellung von Verbindungen der Formel (I), worin R eine Gruppe der Formel: 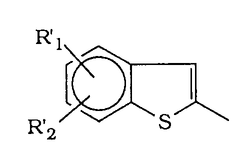 darstellt, in der R'₁ und R'₂, die gleichartig oder verschieden sein können, ein Wasserstoff- oder Halogenatom oder eine niedrigmolekulare Alkylgruppe, eine niedrigmolekulare Alkoxygruppe oder eine Trifluormethylgruppe bedeuten, von deren optischen Isomeren sowie gegebenenfalls von deren Additionssalzen mit einer pharmazeutisch annehmbaren Base oder Säure.

7. Verfahren nach Anspruch 1 zur Herstellung von Verbindungen der Formel (I), worin R eine Gruppe: 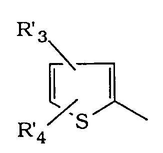 darstellt, worin R'₃ und R'₄, die gleichartig oder verschieden sein können, eine Gruppe ausgewählt aus Wasserstoff, Halogen, niedrigmolekularen Alkylgruppen, niedrigmolekularen Alkoxygruppen oder Trifluormethylgruppen mit der Maßgabe bedeuten, daß, wenn R'₃ ein Wasserstoffatom darstellt, R'₄ kein Wasserstoffatom, Chloratom oder Bromatom oder eine Methylgruppe bedeuten kann, von deren optischen Isomeren sowie gegebenenfalls von deren Additionssalzen mit einer pharmazeutisch annehmbaren Base oder Säure.

8. Verfahren nach Anspruch 1 zur Herstellung von Verbindungen der Formel (I), worin R eine Gruppe: 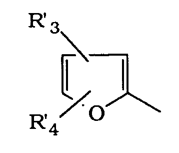 darstellt, worin R'₃ und R'₄, die gleichartig oder verschieden sein können, eine Gruppe ausgewählt aus Wasserstoff, Halogen, niedrigmolekularen Alkylgruppen, niedrigmolekularen Alkoxygruppen oder Trifluormethylgruppen mit der Maßgabe bedeuten, daß, wenn R'₃ ein Wasserstoffatom darstellt, R'₄ kein Wasserstoffatom oder eine Methylgruppe bedeuten kann, von deren optischen Isomeren sowie gegebenenfalls von deren Additionssalzen mit einer pharmazeutisch annehmbaren Base oder Säure.

9. Verfahren nach Anspruch 1 zur Herstellung von Verbindungen der Formel (I), worin R eine Gruppe: 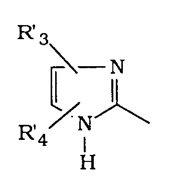 darstellt, worin R'₃ und R'₄, die gleichartig oder verschieden sein können, eine Gruppe ausgewählt aus Wasserstoff, Halogen, niedrigmolekularen Alkylgruppen, niedrigmolekularen Alkoxygruppen und Trifluormethylgruppen bedeuten, von deren optischen Isomeren sowie gegebenenfalls von deren Additionssalzen mit einer pharmazeutisch annehmbaren Base oder Säure.

10. Verfahren nach Anspruch 1 zur Herstellung von Verbindungen der Formel (I), worin R:
- eine aromatische Gruppe mit 6 Gliedern, die gegebenenfalls substituiert ist und in ihrem Kohlenstoffskelett 2 oder 3 Stickstoffatome aufweist,
- eine aromatische Gruppe mit 6 Gliedern, die in ihrem Kohlenstoffskelett 1 bis 3 Stickstoffatome aufweist und mit einem Benzolring verbunden ist, wobei diese beiden Ringe gegebenenfalls substituiert sein können,
- oder einen Ring mit sieben Gliedern, der gesättigt ist oder nicht und in seinem Kohlenstoffskelett 1 oder 2 Stickstoffatome aufweist und gegebenenfalls mit einem Benzolring verbunden ist und gegebenenfalls am stickstoffhaltigen Ring und/oder am Benzolring substituiert ist,
bedeutet,
wobei der Begriff "substituiert" für die damit gekennzeichneten Gruppen bedeutet, daß diese durch eines oder mehrere Halogenatome oder niedrigmolekulare Alkylgruppen oder niedrigmolekulare Alkoxygruppen oder Hydroxylgruppen oder Trifluormethylgruppen oder Aminogruppen oder niedrigmolekulare Alkylaminogruppen oder Nitrogruppen substituiert sein können,
von deren Isomeren sowie von deren Additionssalzen mit einer pharmazeutisch annehmbaren Säure oder Base.

11. Verfahren nach Anspruch 1 zur Herstellung von Verbindungen der Formel (I), worin R eine Indolyl-, Pyrimidinyl-, Pyridazinyl-, 1, 3, 5-Triazinyl-oder Azepinyl-gruppe darstellt, die gegebenenfalls durch eine niedrigmolekulare Alkylgruppe, niedrigmolekulare Alkoxygruppe, eine Trifluormethylgruppe oder durch eines oder mehrere Halogenatome substituiert ist, von deren Isomeren sowie von deren Additionssalzen mit einer pharmazeutisch annehmbaren Säure oder Base.

12. Verfahren nach Anspruch 1 zur Herstellung von 3-(2-Imidazolyl)-4-aminobutansäure, von deren Isomeren sowie von deren Additionssalzen mit einer pharmazeutisch annehmbaren Säure oder Base.

13. Verfahren nach Anspruch 1 zur Herstellung von 3-(2-(5-Isopropyl-benzofuryl))-4-amino-butansäure, von deren Isomeren sowie von deren Additionssalzen mit einer pharmazeutisch annehmbaren Säure oder Base.

14. Verfahren nach Anspruch 1 zur Herstellung von 3-(2-(5-Ethyl-benzofuryl))-4-amino-butansäure, von deren Isomeren sowie von deren Additionssalzen mit einer pharmazeutisch annehmbaren Säure oder Base.

15. Verfahren nach Anspruch 1 zur Herstellung von 3-(2-(5-(1-Methylpropyl)-benzofuryl))-4-amino-butansäure, von deren Isomeren sowie von deren Additionssalzen mit einer pharmazeutisch annehmbaren Säure oder Base.

16. Verfahren nach Anspruch 1 zur Herstellung von 3-(2-(5-Fluor-benzofuryl))-4-amino-butansäure, von deren Isomeren sowie von deren Additionssalzen mit einer pharmazeutisch annehmbaren Säure oder Base.

17. Verfahren nach Anspruch 1 zur Herstellung von 3-(2-(4,5-Dichlor-thienyl))-4-amino-butansäure, von deren Isomeren sowie von deren Additionssalzen mit einer pharmazeutisch annehmbaren Säure oder Base.

18. Verfahren nach Anspruch 1 zur Herstellung von 3-(2-(5-Chlor-benzofuryl))-4-amino-butansäure, von deren Isomeren sowie von deren Additionssalzen mit einer pharmazeutisch annehmbaren Säure oder Base.

19. Verfahren nach Anspruch 1 zur Herstellung von 3-(2-Benzothienyl)-4-amino-butansäure, von deren vonIsomeren sowien deren Additionssalze mit einer pharmazeutisch annehmbaren Säure oder Base.

20. Verfahren nach Anspruch 1 zur Herstellung von 3-(2-(5-Methyl-benzofuryl))-4-amino-butansäure, von deren Isomeren sowie von deren Additionssalzen mit einer pharmazeutisch annehmbaren Säure oder Base.

21. Verfahren nach Anspruch 1 zur Herstellung von 3-(2-(5-Ethoxy-benzofuryl))-4-amino-butansäure, von deren Isomeren sowie von deren Additionssalzen mit einer pharmazeutisch annehmbaren Säure oder Base.

22. Verfahren zur Herstellung der Verbindungen der Formel (II): 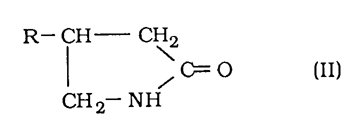 in der R die in Anspruch 1 angegebenen Bedeutungen besitzt,
mit der Maßgabe, daß R:
- keine 2-Benzofurylgruppe, die nichtsubstituiert oder am Benzolkern durch ein Chloratom, ein Bromatom oder eine Methoxygruppe substituiert ist,
- oder keine 2-Benzothienylgruppe darstellt,
von deren Isomeren sowie gegebenenfalls von deren Additionssalzen mit einer Säure oder Base,
**dadurch gekennzeichnet**, daß man
- entweder einen Aldehyd der Formel (V):
R - CHO (V)
in der R die oben angegebenen Bedeutungen besitzt, in einem apolaren aprotisehen Lösungsmittel
mit einem Carboxymethyliden-triphenylphosphoran-Ester der Formel (VI):
(C₆H₅)₃ - P = CH - COOR' (VI)
in der R' eine niedrigmolekulare Alkylgruppe darstellt, kondensiert,
zur Bildung eines Esters der Formel (VII):
R - CH = CH - COOR' (VII)
in der R und R' die oben angegebenen Bedeutungen besitzen,
welchen man in polarem protischem Medium mit Nitromethan in Gegenwart einer starken Base kondensiert zur Bildung einer Verbindung der Formel (VIII): 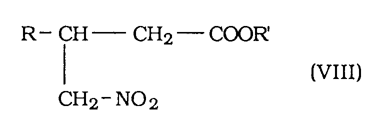 in der R und R' die oben angegebenen Bedeutungen besitzen,
welche in alkoholischem Medium durch Einwirkung von Wasserstoff in Gegenwart des Metallkatalysators zu einer Verbindung der Formel (IX) reduziert wird: 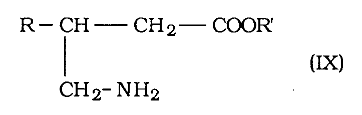 in der R und R' die oben angegebenen Bedeutungen besitzen,
welche durch Erhitzen zu einer Verbindung der Formel (II) cyclisiert wird: 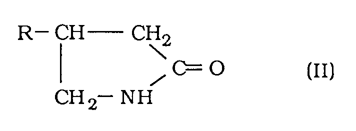 in der R die oben angegebenen Bedeutungen besitzt,
- oder eine Verbindung der Formel (X):
RCOCH₃ (X)
in der R die oben angegebenen Bedeutungen besitzt,
in der Wärme und in Gegenwart von Zink mit einer Verbindung der Formel (XI):
Br - CH₂ - COOA (XI)
worin A eine niedrigmolekulare Alkylgruppe darstellt, behandelt,
so daß man nach der sauren Hydrolyse und der eventuellen Extraktion und Reinigung eine Verbindung der Formel (XII) erhält: 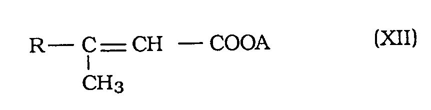 in der R und A die oben angegebenen Bedeutungen besitzen,
welche man mit N-Brom-succinimid behandelt zur Bildung einer Verbindung der Formel (XIII): 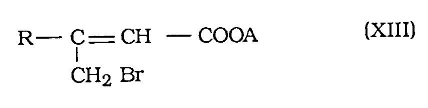 in der R und A die oben angegebenen Bedeutungen besitzen,
welche man mit Ammoniak, vorzugsweise im Überschuß, behandelt zur Bildung einer Verbindung der Formel (XIV): 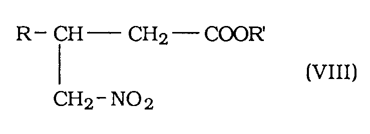 in der R die oben angegebenen Bedeutungen besitzt,
welche man einer katalytischen Hydrierung unterwirft zur Bildung einer Verbindung der Formel (II), wie sie oben definiert worden ist.

23. Verfahren zur Herstellung von pharmazeutischen Zubereitungen enthaltend als Wirkstoff mindestens eine Verbindung hergestellt nach einem der Ansprüche 1 bis 21 in Kombination mit einem oder mehreren inerten, nichttoxisehen, pharmazeutisch annehmbaren Trägermaterialien oder Bindemitteln.

24. Verfahren zur Herstellung von pharmazeutischen Zubereitungen nach Anspruch 23 enthaltend mindestens einen Wirkstoff hergestellt nach einem der Ansprüche 1 bis 21 zur Behandlung von spastischen Störungen und Störungen des Alterns.
